(19) Europäisches Patentamt
European Patent Office
Office européen des brevets

(11) EP 2 918 600 B1

(12) **EUROPEAN PATENT SPECIFICATION**

(45) Date of publication and mention of the grant of the patent:
**24.04.2019 Bulletin 2019/17**

(21) Application number: **13852655.3**

(22) Date of filing: **06.11.2013**

(51) Int Cl.:
*C07K 14/435* (2006.01) *C07K 16/18* (2006.01)
*C12Q 1/02* (2006.01) *G01N 33/15* (2006.01)
*G01N 33/50* (2006.01) *G01N 33/53* (2006.01)
*G01N 33/68* (2006.01)

(86) International application number:
**PCT/JP2013/080614**

(87) International publication number:
**WO 2014/073708 (15.05.2014 Gazette 2014/20)**

(54) **NOVEL PEPTIDE AND USE THEREOF**

NEUARTIGES PEPTID UND VERWENDUNG DAVON

NOUVEAU PEPTIDE ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2012 JP 2012244752**
**22.02.2013 JP 2013033651**

(43) Date of publication of application:
**16.09.2015 Bulletin 2015/38**

(73) Proprietor: **University of Miyazaki**
**Miyazaki-shi**
**Miyazaki 889-2192 (JP)**

(72) Inventors:
- **KITAMURA, Kazuo**
**Miyazaki-shi**
**Miyazaki 889-1692 (JP)**
- **NAGATA, Sayaka**
**Miyazaki-shi**
**Miyazaki 889-1692 (JP)**
- **ASADA, Yujiro**
**Miyazaki-shi**
**Miyazaki 889-1692 (JP)**

(74) Representative: **Vossius & Partner**
**Patentanwälte Rechtsanwälte mbB**
**Siebertstrasse 3**
**81675 München (DE)**

(56) References cited:
**WO-A2-2005/114221 WO-A2-2007/047796**
**JP-A- 2010 500 568**

- **CAMPBELL D J ET AL: "Processing of rat and human angiotensinogen precursors by microsomal membranes", MOLECULAR AND CELLULAR ENDOCRINOLOGY, ELSEVIER IRELAND LTD, IE, vol. 43, no. 1, 1 November 1985 (1985-11-01), pages 31-40, XP025218670, ISSN: 0303-7207, DOI: 10.1016/0303-7207(85)90039-5 [retrieved on 1985-11-01]**
- **SUZAKI Y ET AL: "Quantification of human angiotensinogen by a novel sandwich ELISA", PEPTIDES, ELSEVIER, AMSTERDAM, NL, vol. 27, no. 11, 1 November 2006 (2006-11-01), pages 3000-3002, XP027957605, ISSN: 0196-9781 [retrieved on 2006-11-01]**
- **KOBORI H ET AL: "Young Scholars Award Lecture: Intratubular Angiotensinogen in Hypertension and Kidney Diseases", AMERICAN JOURNAL OF HYPERTENSION, NATURE PUBLISHING GROUP, UNITED STATES, vol. 19, no. 5, 1 May 2006 (2006-05-01), pages 541-550, XP024903920, ISSN: 0895-7061, DOI: 10.1016/J.AMJHYPER.2005.11.014 [retrieved on 2006-05-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 918 600 B1

- NAGATA S ET AL: "Isolation and identification of proangiotensin-12, a possible component of the renin-angiotensin system", BIOCHEMICAL AND BIOPHYSICAL RESEARCH COMMUNICATIONS, ACADEMIC PRESS INC. ORLANDO, FL, US, vol. 350, no. 4, 1 December 2006 (2006-12-01), pages 1026-1031, XP024924751, ISSN: 0006-291X, DOI: 10.1016/J.BBRC.2006.09.146 [retrieved on 2006-12-01]
- HUANG XIAO R ET AL: "Chymase is upregulated in diabetic nephropathy: Implications for an alternative pathway of angiotensin II-mediated diabetic renal and vascular disease", JOURNAL OF THE AMERICAN SOCIETY OF NEPHROLOGY, WILLIAMS AND WILKINS, BALTIMORE, MD, US, vol. 14, no. 7, 1 July 2003 (2003-07-01), pages 1738-1747, XP002524027, ISSN: 1046-6673, DOI: 10.1097/01.ASN.0000071512.93927.4E
- NAGATA SAYAKA ET AL: "Big angiotensin-25: A novel glycosylated angiotensin-related peptide isolated from human urine", BIOCHEMICAL AND BIOPHYSICAL RESEARCH COMMUNICATIONS, vol. 441, no. 4, 5 November 2013 (2013-11-05), pages 757-762, XP028787941, ISSN: 0006-291X, DOI: 10.1016/J.BBRC.2013.10.124
- NAGATA S. ET AL.: 'Big angiotensin-25: A novel glycosylated angiotensin-related peptide isolated from human urine' BIOCHEM. BIOPHYS. RES. COMMUN. vol. 441, no. 4, 05 November 2013, pages 757 - 762, XP028787941
- NAGATA S. ET AL.: 'Isolation and identificatior of proangiotensin-12, a possible component of the renin-angiotensin system' BIOCHEM. BIOPHYS. RES. COMMUN. vol. 350, no. 4, 2006, pages 1026 - 1031, XP024924751
- AHMAD S. ET AL.: 'Chymase-dependent generation of angiotensin II from angiotensin-(1-12) in human atrial tissue' PLOS ONE vol. 6, no. 12, 2011, page E28501, XP055254798
- WESTWOOD BM. ET AL.: 'Divergent pathways for the angiotensin-(1-12) metabolism in the rat circulation and kidney' PEPTIDES vol. 35, no. 2, June 2012, pages 190 - 195, XP028487414

## Description

Technical Field

The present invention relates to a novel peptide associated with the renin-angiotensin system and applications of such peptide for diagnosis of a renal disease or a cardiovascular disease.

Background Art

The renin-angiotensin system (RAS) plays a key role in the regulation of blood pressure and fluid balance in the body, and it is deeply involved with the development and advancement of renal and cardiovascular diseases. As population aging advances, the number of people afflicted with such diseases is increasing every year. At present, accordingly, it is imperative to use agents that suppress the RAS (e.g., angiotensin-receptor antagonists or angiotensin-converting enzyme inhibitors) or to evaluate the RAS through biochemical examination for the treatment and/or diagnosis of a renal disease or a cardiovascular disease.

The mechanism of the RAS in the circulating blood is as described below. At the outset, angiotensinogen comprising 452 amino acids primarily synthesized in the liver is converted into angiotensin I (Ang I) comprising 10 amino acids located at the N-terminus of angiotensinogen by renin secreted from the kidneys. Subsequently, angiotensin I (Ang I) is converted into angiotensin II (Ang II) by the angiotensin converting enzyme (ACE) existing in the pulmonary capillaries. Angiotensin II (Ang II) binds to the angiotensin II receptor and accelerates blood vessel constriction, aldosterone secretion, and sodium reabsorption to thereby induce blood pressure elevation.

While RAS activity in the blood has heretofore drawn attention as a vasopressor system as described above, the existence of a tissue RAS produced in local tissue has been pointed out in recent years, and the renin-independent angiotensin production pathway has also been pointed out. It has been found out that, independent of the RAS in the blood, the tissue RAS is involved in damages to various organs, as well as blood pressure regulation. That is, there are various theories regarding the angiotensin production pathway *in vivo,* and the production mechanism and the role thereof remain unknown. If the renin-independent angiotensin II production pathway is elucidated, accordingly, the possibility of development of novel pharmaceutical products can be expected.

Moreover, the processing of rat and human angiotensinogen precursors by microsomal membranes has previously been described (Non-Patent Document 1). Further, the quantification of human angiotensinogen by a novel sandwich ELISA has recently been described (Non-Patent Document 2). In addition, intratubular angiotensinogen in hypertension and kidney diseases has previously been described (Non-Patent Document 3).

The present inventors discovered pro-angiotensin-12 from rat tissue (the small intestine). Pro-angiotensin-12 comprises 12 amino acids (Asp-Arg-Val-Tyr-Ile-His-Pro-Phe-His-Leu-Leu-Tyr) extending further toward the C-terminus by 2 amino acids than angiotensin I, and it is a peptide equivalent to rat angiotensinogen (1-12). It has also been demonstrated that a polypeptide equivalent to human angiotensinogen (1-12) is present in human tissue (i.e., the placenta) (Patent Document 1). In recent years, angiotensin (1-7), angiotensin III, and angiotensin IV, which are angiotensin II-derived peptides, have also been reported (Non-Patent Documents 1 and 2).

Up to the present, RAS components, such as angiotensinogen, angiotensin I, angiotensin II, and renin, aldosterone that is accelerated to produce by angiotensin II, and other substances have been used as biomarkers of a cardiovascular disease or organ damage caused by high blood pressure on the basis of RAS activity *in vivo.* If a new RAS component is discovered, accordingly, it can be expected to serve as a new biomarker of the diseases or damages described above. Meanwhile, urine albumin is often used as a renal disease biomarker. In recent years, also, it has been reported that the urine angiotensinogen level becomes elevated because of nephropathy, high blood pressure, salt loading, or other causes, and urine angiotensinogen is expected to serve as a novel renal disease biomarker. However, the molecular weight of albumin is as large as 66,000, and that of angiotensinogen is as large as about 50,000. By the time it is excreted in urine, accordingly, glomerular disorder has already advanced to a significant extent. Accordingly, discovery of a biomarker leading to early detection of renal disorders has been awaited.

Prior Art Documents

Patent Documents

Patent Document 1: JP 2008-007482 A

Non-Patent Documents

Non-Patent Document 1: Science 184, 1974, 994-996
Non-Patent Document 2: Am. J. Physiol. 268, 1995, L302-308

Non-Patent Document 1: Molecular and Cellular Endocrinology, 43(1), 1985, 31-34
Non-Patent Document 2: Peptides, 27(11), 3000-3002
Non-Patent Document 3: American Journal of Hypertension and Kidney Diseases, 19(5), 541-550

Summary of the Invention

Discovery of a novel component of the renin-angiotensin system (RAS) associated with the development of a renal disease or a cardiovascular disease or a novel angiotensin production pathway as described above would lead to increased potential for the development of a novel pharmaceutical product.

Accordingly, an object of the present invention is to discover a novel RAS component and to provide the same as a novel biomarker of a renal disease or a cardiovascular disease.

In order to achieve the aforementioned object, the present inventors searched for a novel RAS component *in vivo* via radioimmunoassay (RIA) that specifically recognizes an area in the vicinity of the N-terminus of angiotensinogen. As a result, they discovered a novel peptide in human urine. They elucidated the following: such novel peptide has a characteristic structure composed of angiotensin II at the N-terminus and 25 amino acids comprising an N-linked glycan and cysteine (Cys) added thereto, the novel peptide is localized specifically in glomerular epithelial cells (podocytes), the concentration thereof is elevated in urine of a patient with a renal disorder associated with diabetes, and the ratio of peptide to creatinine in urine is positively correlated with the percentage of urine protein or estimated Glomerular Filtration Rate (eGFR). Since such novel peptide was considered to be associated with angiotensin I or angiotensin II, it was designated "Big angiotensin-25" (BAng-25).

In addition, the present inventors varied the presence or absence and the types of glycosyl chain added to BAng-25 and investigated the effects thereof on renin and on chymase. As a result, BAng-25 was found to be a chymase-specific substrate, although it would not serve as a renin substrate. Thus, BAng-25 was found to be a novel substance associated with the renin-independent angiotensin II production pathway. Further, BAng-25 was found to be present at high concentrations in the heart, the blood vessels, the nervous and endocrine tissues, and the genital tissue, as well as in the kidneys. Thus, BAng-25 was found to be a novel target molecule of renal and cardiovascular diseases, nervous and endocrine diseases, and genital diseases. The present invention has been completed based on such findings.

The present invention relates to the embodiments as characterized in the claims. Thus, it relates to the following items.

1. A peptide consisting of the amino acid sequence as shown in SEQ ID NO: 1 which has a disulfide bond involving the cysteine residue at position 18 of the amino acid sequence as shown in SEQ ID NO: 1 and another cysteine or an amino acid sequence derived therefrom by deletion, substitution, or addition of 1 to 5 amino acids other than the cysteine residue at position 18 and other than the asparagine residue at position 14 and an N-linked glycan added thereto at the asparagine residue at position 14, wherein the peptide is resistant against renin and reactive with chymase.

2. The peptide according to item 1, wherein the N-linked glycan may optionally comprise sialic acid bound thereto at the non-reducing terminus and the glycosyl chain is constituted by sugars, including galactose, N-acetylglucosamine, and mannose.

3. The peptide according to item 1 or 2, wherein the N-linked glycan has a structure represented by Formula (I), (II), or (III):

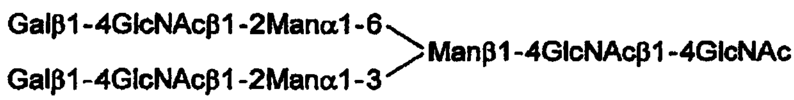 (I)

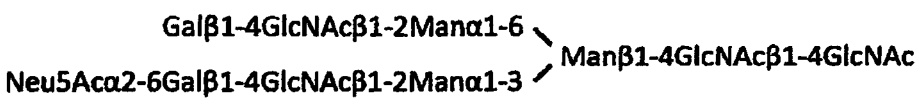 (II)

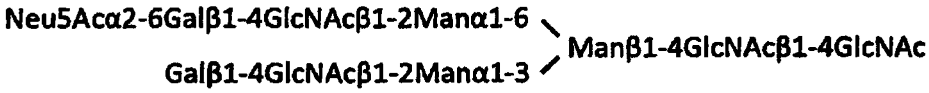
(III)

wherein, in the above Formula (I), (II), or (III), Gal represents galactose; Man represents mannose; GlcNAc represents N-acetylglucosamine; and Neu5Ac represents N-acetylneuraminic acid.

4. Use of the peptide according to any of items 1 to 3 as a diagnostic marker for diagnosing a diabetic nephropathy or a cardiovascular disease in vitro.

5. In vitro use of the peptide according to any of items 1 to 3 as a chymase substrate.

6. A test method for diagnosis of diabetic nephropathy or a cardiovascular disease comprising measuring the amount of the peptide according to any of items 1 to 3 in a biological sample obtained from a subject.

7. A method for evaluating effects of treating a diabetic nephropathy or a cardiovascular disease comprising investigating a change in the amount of the peptide according to any of items 1 to 3 in biological samples obtained from a subject before and after the treatment.

8. The method according to item 6 or 7, wherein the biological sample is urine.

9. A method for screening for a therapeutic agent for a diabetic nephropathy or a cardiovascular disease comprising bringing a test substance into contact with mammalian-derived glomerular epithelial cells or administering a test substance to a non-human mammalian and measuring the amount of the peptide according to any of items 1 to 3 in the cells or urine to investigate a change in the amount of the peptide.

10. The method according to any of items 6 to 9, wherein the amount of the peptide is measured via immunological assay using an antibody that binds specifically to a peptide consisting of residues 18 to 25 of the amino acid sequence as shown in SEQ ID NO:1.

11. An antibody that binds specifically to a peptide consisting of residues 18 to 25 of the amino acid sequence as shown in SEQ ID NO:1.

12. An agent for diagnosis of a diabetic nephropathy or a cardiovascular disease comprising the antibody that binds specifically to a peptide consisting of residues 18 to 25 of the amino acid sequence as shown in SEQ ID NO:1.

13. A kit for diagnosis of a diabetic nephropathy or a cardiovascular disease comprising the antibody that binds specifically to a peptide consisting of residues 18 to 25 of the amino acid sequence as shown in SEQ ID NO:1.

Moreover, in the context of the present invention, there is disclosed the following.

(1) A peptide consisting of the amino acid sequence as shown in SEQ ID NO: 1 or an amino acid sequence derived therefrom by deletion, substitution, or addition of one to several amino acids other than the asparagine residue at position 14 and an N-linked glycan added thereto at the asparagine residue at position 14.
(2) The peptide according to (1), wherein the N-linked glycan may optionally comprise sialic acid bound thereto at the non-reducing terminus and the glycosyl chain is constituted by sugars, including galactose, N-acetylglucosamine, and mannose.
(3) The peptide according to (1) or (2), wherein the N-linked glycan has a structure represented by Formula (I), (II), or (III):

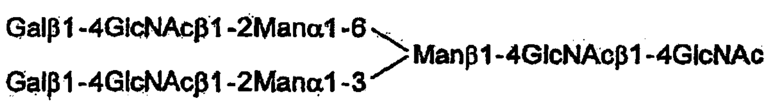
(I)

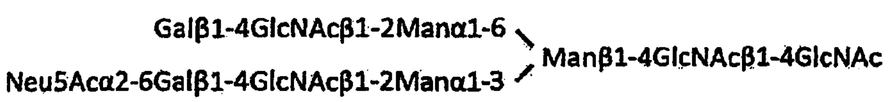
(II)

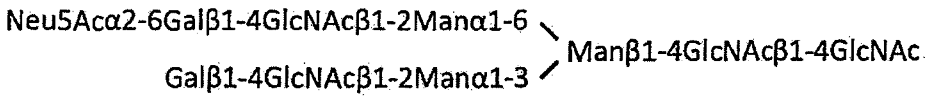

(III)

wherein, in the above Formula (I), (II), or (III), Gal represents galactose; Man represents mannose; GlcNAc represents N-acetylglucosamine; and Neu5Ac represents N-acetylneuraminic acid.

(4) The peptide according to any of (1) to (3), which has a disulfide bond involving cysteine residue at position 18 of the amino acid sequence as shown in SEQ ID NO: 1 or an amino acid sequence derived therefrom by deletion, substitution, or addition of one to several amino acids other than the asparagine residue at position 14.

(5) The peptide according to any of (1) to (4), which is a diagnostic marker of a renal disease or a cardiovascular disease.

(6) The peptide according to any of (1) to (4), which serves as a chymase substrate.

(7) A test method for diagnosis of a renal disease or a cardiovascular disease comprising measuring the amount of the peptide according to any of (1) to (4) in a biological sample obtained from a subject.

(8) A method for evaluating effects of treating a renal disease or a cardiovascular disease comprising investigating a change in the amount of the peptide according to any of (1) to (4) in biological samples obtained from a subject before and after the treatment.

(9) The method according to (7) or (8), wherein the biological sample is urine.

(10) A method for screening for a therapeutic agent for a renal disease or a cardiovascular disease comprising bringing a test substance into contact with mammalian-derived glomerular epithelial cells or administering a test substance to a non-human mammalian and measuring the amount of the peptide according to any of (1) to (4) in the cells or urine to investigate a change in the amount of the peptide.

(11) The method according to any of (7) to (10), wherein the amount of the peptide is measured via immunological assay using an antibody that binds specifically to the peptide.

(12) An antibody that binds specifically to the peptide according to any of (1) to (4).

(13) An agent for diagnosis of a renal disease or a cardiovascular disease comprising the antibody that binds specifically to the peptide according to any of (1) to (4).

(14) A kit for diagnosis of a renal disease or a cardiovascular disease comprising the antibody that binds specifically to the peptide according to any of (1) to (4).

This patent application claims priority from Japanese Patent Application No. 2012-244752 filed on November 6, 2012 and Japanese Patent Application No. 2013-033651 filed on February 22, 2013.

Effects of the Invention

The present invention provides big angiotensin-25 (BAng-25), which is a novel peptide associated with the renin-independent angiotensin II production pathway. BAng-25 is localized in renal tissue (podocytes, in particular), and the concentration thereof in urine of a patient with diabetes associated with proteinuria is elevated to a significant extent. Thus, BAng-25 is useful as a biomarker of nephropathy or diabetic nephropathy. With the use of BAng-25, accordingly, renal diseases and cardiovascular diseases can be diagnosed at the early stage in a simple manner without imposing burdens on a subject. Also, BAng-25 can be used for verification of therapeutic effects on patients with the diseases described above, follow-up, and screening of novel therapeutic agents. Further, BAng-25 is present at high concentrations in the nervous and endocrine tissues such as adrenal medulla, Langerhans' islet of the pancreas, and the nerve plexus of the intestinal tract, and genital tissue such as the placenta and testicle, as well as in organs such as the kidneys and the heart. Accordingly, BAng-25 may be able to serve as a novel target molecule of nervous, endocrine, or genital diseases, in addition to renal diseases and cardiovascular diseases, such as the heart disease.

In addition, a glycosyl chain structure that was added to BAng-25 is resistant against renin, it is reactive with chymase, and it is capable of regulation of angiotensin production. Accordingly, modification with a glycosyl chain can be a novel means for regulation of angiotensin production. Since vaccine therapy against angiotensin or an angiotensin receptor has been attempted in recent years, use of BAng-25 as an RAS vaccine is expected to result in effects of renal protection or blood pressure reduction.

Brief Description of the Drawings

Fig. 1A shows the results obtained by subjecting a human urine sample to gel filtration and measuring the amount

of immunoreactive N-terminal Ang II of each fraction via radioimmunoassay. (An arrow indicates a fraction containing immunoreactive N-terminal Ang II.) Fig. 1B shows the final purification of the immunoreactive fraction via RP-HPLC. Fig. 1C shows the structural analysis of the purified peptide using a quadrupole time-of-flight mass spectrometer. Fig. ID shows the amino acid analysis of the purified peptide using a protein sequencer.

Fig. 2A shows the primary structural analysis of the N-linked glycan contained in the purified peptide. Fig. 2B shows the secondary structural analysis of the N-linked glycan contained in the purified peptide. Fig. 2C shows the structure of the purified peptide (big angiotensin-25). (That is, the peptide comprises 25 amino acids and the N-linked glycan and Cys added to Asn at position 14 and an amino acid at position 18, respectively.)

Fig. 3 shows the structure of big angiotensin-25 with a glycosyl chain (BAng-25), that of big angiotensin-25 without a glycosyl chain (Non-glycosyl chain BAng-25), and that of big angiotensin-25 with GlcNAc substitution (BAng-25+GlcNAc).

Fig. 4A and Fig. 4B each show the results of HPLC analysis on reactivity of renin with big angiotensin-25 with a glycosyl chain (BAng-25) (Fig. 4A: in the absence of renin; Fig. 4B: 100 U renin). Fig. 4C and Fig. 4D each show the results of HPLC analysis on reactivity of renin with big angiotensin-25 without a glycosyl chain (Non-glycosyl chain BAng-25) (Fig. 4C: in the absence of renin; Fig. 4D: 100 U renin). Fig. 4E and Fig. 4F each show the results of HPLC analysis on reactivity of renin with big angiotensin-25 with GlcNAc substitution (BAng-25+GlcNAc) (Fig. 4E: in the absence of renin; Fig. 4F: 100 U renin).

Fig. 5 shows the amounts of Ang I produced after big angiotensin-25 without a glycosyl chain (Non-glycosyl chain BAng-25) and big angiotensin-25 with a glycosyl chain (BAng-25) were each subjected to reactions with 20 U renin for 24 hours (black circle: Non-glycosyl chain BAng-25; white circle: BAng-25).

Fig. 6A and Fig. 6B each show the results of HPLC analysis on reactivity of chymase with big angiotensin-25 with a glycosyl chain (BAng-25) (Fig. 6A: in the absence of chymase; Fig. 6B: 43.4 mU chymase). Fig. 6C and Fig. 6D each show the results of HPLC analysis on reactivity of chymase with big angiotensin-25 without a glycosyl chain (Non-glycosyl chain BAng-25) (Fig. 6C: in the absence of chymase; Fig. 6D: 43.4 mU chymase). Fig. 6E and Fig. 6F each show the results of HPLC analysis on reactivity of chymase with big angiotensin-25 with GlcNAc substitution (BAng-25+GlcNAc) (Fig. 6E: in the absence of chymase; Fig. 6F: 43.4 mU chymase).

Fig. 7 shows the amounts of Ang II produced after big angiotensin-25 with a glycosyl chain (BAng-25), big angiotensin-25 without a glycosyl chain (Non-glycosyl chain BAng-25), and big angiotensin-25 with GlcNAc substitution (BAng-25+GlcNAc) were each subjected to reactions with 20 U chymase for 24 hours (rhombus: BAng-25; square: Non-glycosyl chain BAng-25; triangle: BAng-25+GlcNAc).

Fig. 8A shows the immunostaining of big angiotensin-25 in the kidneys (left: scale bar: 100 μm; right: scale bar: 50 μm). Fig. 8B shows the correlation between the ratio of big angiotensin-25 to urine creatinine (BAng-25/UCre) and the estimated Glomerular Filtration Rate (eGFR). Fig. 8C shows the correlation between the ratio of big angiotensin-25 to urine creatinine (BAng-25/UCre) and the proteinuria of a patient with diabetes (Upro(-): proteinuria-negative; Upro(+): proteinuria-positive). Fig. 8D shows the correlation between the ratio of big angiotensin-25 to urine creatinine (BAng-25/UCre) and proteinuria (creatinine correction).

Fig. 9 shows the immunostaining of big angiotensin-25 in the heart (the left ventricle).

Fig. 10 shows the immunostaining of big angiotensin-25 in the stratified squamous epithelium of the esophagus (the upper panel) and in the esophageal smooth muscle cells of the esophagus (the lower panel).

Fig. 11 shows the immunostaining of big angiotensin-25 in the adrenal gland (the upper panel) and in the adrenal medulla (the lower panel).

Fig. 12 shows the immunostaining of big angiotensin-25 in the islets of Langerhans.

Fig. 13 shows the immunostaining of big angiotensin-25 in the gastrointestinal endocrine cells of the stomach (the upper panel), in the nerve fibers within the submucosa layer of the stomach (the lower left panel), and in Auerbach's plexus of the stomach (the lower right panel).

Fig. 14 shows the immunostaining of big angiotensin-25 in the gastrointestinal endocrine cells of the large intestine.

Fig. 15 shows the immunostaining of big angiotensin-25 in the extravillous trophoblast cells of the placenta (the upper panel) and in the decidua of the placenta (the lower panel).

Fig. 16 shows the immunostaining of big angiotensin-25 in the salpinx.

Fig. 17 shows the immunostaining of big angiotensin-25 in the testicle.

## Embodiments for Carrying out the Invention

### 1. Big angiotensin-25 (BAng-25)

The novel peptide associated with the renin-angiotensin system of the present invention; i.e., big angiotensin-25 (hereafter, it may be simply referred to as "BAng-25"), has a characteristic structure such that it consists of the following amino acid sequence: Asp-Arg-Val-Tyr-Ile-His-Pro-Phe-His-Leu-Val-Ile-His-Asn-Glu-Ser-Thr-Cys-Glu-Gln-Leu-Ala-Lys-Ala-Asn (SEQ ID NO: 1), and an N-linked glycan added thereto at the asparagine residue at position 14.

BAng-25 of the present invention has a disulfide bond involving the cysteine residue at position 18 of the amino acid sequence as shown in SEQ ID NO: 1. Typically, cysteine is added to the cysteine residue via a disulfide bond. In such a case, a residue at position 18 of the amino acid sequence as shown in SEQ ID NO: 1 is cystine (Cys-Cys).

Angiotensinogen is immediately converted into angiotensin I with the aid of renin in the blood. In contrast, renin does not act on BAng-25, which is a peptide consisting of the amino acid sequence as shown in SEQ ID NO: 1 and an N-linked glycan added thereto at the asparagine residue at position 14, if an N-linked glycan is added thereto, and angiotensin I is not produced, as described in the examples below. In addition, BAng-25 indicates unique reactivity with renin, such that renin acts BAng-25 to which no N-linked glycan has been added, and angiotensin I is immediately produced. Regardless of the presence or absence of a glycosyl chain, chymase acts on BAng-25, and angiotensin II is then produced. Specifically, BAng-25 would not serve as a renin substrate, but it would serve as a chymase-specific substrate.

As long as BAng-25 of the present invention is resistant against renin and reactive with chymase, accordingly, BAng-25 may be a modified peptide thereof. For example, such modified peptide is a peptide comprising an amino acid sequence derived from the amino acid sequence as shown in SEQ ID NO: 1 by deletion, substitution, insertion, or addition of one to several amino acids other than the asparagine residue at position 14 and the cysteine residue at position 18. The term "one to several" used herein refers to 1 to 5 (homology to the amino acid sequence as shown in SEQ ID NO: 1: 80% or higher, preferably 90% or higher, more preferably 95% or higher, and further preferably 99% or higher). The term "resistance [or resistant] against renin" used herein refers to a situation in which there is no or little reactivity with renin. More specifically, angiotensin I is not produced from a target substance (a substrate) with the aid of renin, or the speed of angiotensin I production is slow and the reaction is less likely to proceed. The speed of renin-substrate reaction can be evaluated on the basis of the Km value according to the enzyme kinetics. As the Km value increases, the reactivity becomes poorer. The Km value of a representative renin substrate; i.e., angiotensinogen, may be compared with that of a target substance. If the Km value of the target substance is 50 times or more as great, and preferably about 80 times or more as great, the reactivity of the target substance with renin can be evaluated as being poor.

When amino acid deletion is intended, an arbitrary amino acid other than asparagine at position 14 and other than cysteine at position 18 is selected and deleted from the amino acid sequence as shown in SEQ ID NO:1. When amino acid addition is intended, one to several amino acids are added to the N- or C-terminus of the amino acid sequence as shown in SEQ ID NO: 1. An example of amino acid substitution is conservative amino acid substitution. The term "conservative amino acid substitution" refers to substitution between amino acids having similar properties in terms of, for example, structure, electrical properties, polarity, or hydrophobicity. Such properties can be classified in terms of, for examine, amino acid side chain similarity. Examples of amino acids comprising basic side chains include lysine, arginine, and histidine. Examples of amino acids comprising acidic side chains include aspartic acid and glutamic acid. Examples of amino acids comprising uncharged polar side chains include glycine, asparagine, glutamic acid, serine, threonine, tyrosine, and cysteine. Examples of amino acids comprising hydrophobic side chains include alanine, valine, leucine, isoleucine, proline, phenylalanine, and methionine. Examples of amino acids comprising branched side chains include threonine, valine, leucine, and isoleucine. Examples of amino acids comprising aromatic side chains include tyrosine, tryptophan, phenylalanine, and histidine.

An N-linked glycan in the peptide of the present invention may comprise sialic acid bound thereto at the non-reducing terminus. The glycosyl chain is constituted by galactose (Gal), N-acetylglucosamine (GlcNAc), and mannose (Man). It may comprise glucose (Glc), fucose (Fuc), N-acetylgalactosamine (GalNAc), glucuronic acid (GlcA), iduronic acid (IdoA), xylose (Xyl), or galacturonic acid (GlcA), provided that the peptide comprising the amino acid sequence as shown in SEQ ID NO: 1 becomes resistant against renin as a result of glycosyl chain modification. When a glycosyl chain is branched, the number of branching is typically 2, and it may be 3 or more.

An example of the N-linked glycan is preferably one represented by any of Formula (I), (II), or (III) below:

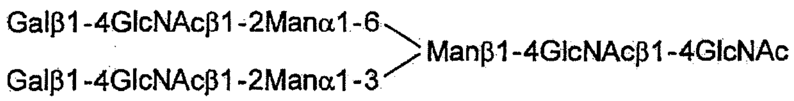

(I)

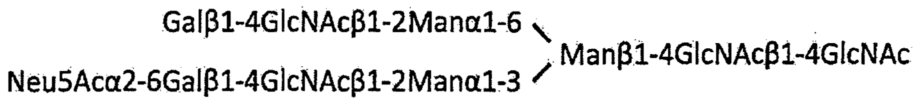

(II)

Neu5Acα2-6Galβ1-4GlcNAcβ1-2Manα1-6
Manβ1-4GlcNAcβ1-4GlcNAc (III)
Galβ1-4GlcNAcβ1-2Manα1-3

wherein, in the above Formula (I), (II), or (III), Gal represents galactose; Man represents mannose; GlcNAc represents N-acetylglucosamine; and Neu5Ac represents N-acetylneuraminic acid.

The peptide of the present invention comprising the N-linked glycan added thereto can be produced by a conventional technique, such as the Convergent method, the Stepwise method, or the Chemo-enzymatic method. According to the Convergent method, a glycosyl chain portion and a peptide portion are separately synthesized, and these portions are condensed, so as to synthesize a glycosylated peptide. According to the Stepwise method, peptide solid phase synthesis is carried out using a sugar amino acid (e.g., glycosyl asparagine in which N-acetylglucosamine is bound to asparagine), so as to extend a peptide and synthesize a glycosylated peptide. According to the Chemo-enzymatic method, a glycosylated peptide comprising N-acetylglucosamine is synthesized, the resultant is designated as a glycosyl chain receptor, various naturally occurring N-linked glycosylated proteins are used as glycosyl chain donors, and a glycosyl chain is transferred and added to the glycosyl chain receptor via a glycosyl chain conversion reaction from a glycosyl chain donor with the aid of endo-β-N-acetylglucosaminidase (e.g., endo-M) to synthesize a new glycosylated peptide.

In addition, BAng-25 of the present invention may optionally be in the form of a salt, and it may preferably be in the form of a biologically acceptable acid addition salt. Examples of such salts include: salts of inorganic acids, such as chloric acid, phosphoric acid, hydrobromic acid, and sulfuric acid; and salts of organic acids, such as acetic acid, formic acid, propionic acid, fumaric acid, maleic acid, succinic acid, tartaric acid, citric acid, malic acid, oxalic acid, benzoic acid, methanesulfonic acid, and benzelsufone.

2. Diagnostic applications of big angiotensin-25 (BAng-25)

As described in the examples below, big angiotensin-25 (BAng-25) is localized specifically in glomerular epithelial cells (podocytes), the urine BAng-25 level and the ratio of urine BAng-25 to creatinine (the urine BAng-25/creatinine ratio) are significantly elevated in patients with diabetes associated with proteinuria and patients with severe nephropathy, and the urine BAng-25/creatinine ratio is positively correlated with proteinuria or the estimated Glomerular Filtration Rate (eGFR). Thus, BAng-25 can be used as a diagnostic marker of a renal disease, such as diabetic nephropathy. Since BAng-25 is an RAS-associated peptide, BAng-25 can serve as a diagnostic marker of a cardiovascular disease caused by hypertension, as can other RAS-associated peptides, such as angiotensin I (Ang I) and angiotensin II (Ang II).

Accordingly, the present invention provides a test method for diagnosis of a diabetic nephropathy or a cardiovascular disease comprising measuring the amount of BAng-25 in a biological sample obtained from a subject.

In the present invention, the "subject" is not particularly limited, as long as it is a mammalian, with a human being particularly preferable. Examples of subjects other than humans include non-human primates, such as monkeys and chimpanzees, and other mammalians, such as dogs, cows, mice, rats, and guinea pigs.

The "biological sample" is not particularly limited, provided that it is body fluid or tissue containing BAng-25. Examples of body fluid include urine, blood, sweat, saliva, spinal fluid, ascites, and lymph. Examples of tissue include tissues derived from the kidneys, the heart, the blood vessels, the brain, the adrenal gland, the pancreas, the ovary, the placenta, and the testicle, gastric endocrine cells, and adipocytes. Urine, blood, and renal tissue are particularly preferable. A urine or blood sample can be prepared without imposing burdens on a subject, and it is often used as a biological sample. Accordingly, use of a urine or blood sample is particularly preferable. A urine sample is further preferable since a biological sample can be obtained in a non-invasive manner.

According to the method of the present invention, the "test for diagnosis" comprises measuring the amount of BAng-25, comparing the sample with a control sample, according to need, and acquiring the data necessary for diagnosis. The amount of BAng-25 may be measured by determining the absolute amount based on a comparison with the standard sample. However, it is not always necessary to determine the absolute amount of BAng-25, and it is sufficient if the relative amount of BAng-25 to that of the control sample is determined.

The term "diagnosis" used in the present invention typically refers to determination as to whether or not a subject is afflicted with a renal disease or a cardiovascular disease ("diagnosis" in a limited sense), although "diagnosis" is not limited thereto. In the present invention, determination of the severity of such a disease, determination of therapeutic effects on such a disease, and determination of the risk of a disease being a recurring disease after treatment are within the scope of "diagnosis" ("diagnosis" in a broad sense).

Examples of renal diseases that can be diagnosed by the method as disclosed herein include diabetic nephropathy, benign nephrosclerosis, focal glomerular sclerosis, membranous nephropathy, minimal change nephrotic syndrome, membranoproliferative glomerulonephritis, rapidly progressive glomerulonephritis, mesangial proliferative nephritis, IgA nephropathy, lupus nephritis, acute nephritis, chronic nephritis (chronic glomerulonephritis), acute renal failure, chronic renal failure, hematuria, proteinuria, electrolyte abnormality, and malignant hypertension. Examples of cardiovascular diseases include arteriosclerosis, stenocardia, heart infarction, arrhythmia, valvular disease, cardiac incompetence, and cerebral apoplexy caused by hypertension.

BAng-25 can be assayed via conventional assay techniques, such as immunological assay or mass analysis (e.g., LC/MS, MS/MS, or LC/MS/MS).

Immunological assay is carried out with the use of an antibody capable of binding specifically to BAng-25. As an antibody capable of binding specifically to BAng-25, an antibody prepared in accordance with a conventional technique using as an antigen a peptide comprising BAng-25 or a partial sequence of BAng-25 can be preferably used. Since the N-terminus of BAng-25 is common to that of Ang I and Ang II, an antibody induced with the use of BAng-25 as an antigen may bind to Ang I and Ang II. In such a case, it is preferable that the BAng-25-specific antibody be purified using an affinity column using a peptide comprising a partial sequence including the C-terminus of BAng-25 (e.g., a peptide comprising residues 18 to 25 of BAng-25). Also, a peptide comprising the partial sequence containing the C-terminus of BAng-25 may be used as an antigen to prepare a BAng-25-specific antibody.

A polyclonal or monoclonal antibody may be used. An antibody fragment can be used, provided that it can bind specifically to BAng-25. Examples of antibody fragments include Fab fragments, F(ab')2 fragments, and single-chain antibody fragments (scFv).

A monoclonal antibody can be prepared, for example, in the manner described below.

The antigen described above is administered to an animal by itself or together with a carrier or a diluent at a site at which an antibody can be produced upon antigen administration. In order to enhance the capacity for antibody production, complete Freund's adjuvant or incomplete Freund's adjuvant may also be administered. Examples of animals to be used include mammalians, such as monkeys, rabbits, dogs, guinea pigs, mice, rats, sheep, and goats, with mice being preferable. The antibody titer in the antiserum can be measured in accordance with a conventional technique.

Animals in which sufficient antibody titers were detected are selected from among animals immunized with antigens, the spleen or lymph nodes are extracted therefrom 2 to 5 days after the final immunization, and antibody-producing cells contained therein are fused to myeloma cells. Thus, monoclonal antibody-producing hybridomas can be prepared. Cell fusion can be carried out in accordance with a known technique, such as the method described in Nature 256: 495, 1975. An example of a cell fusion promoter is polyethylene glycol (PEG). Examples of myeloma cells include NS-1, P3U1, and SP2/0.

Monoclonal antibodies can be selected by a known method or a method in accordance therewith. In general, antibody selection can be carried out in animal cell culture medium supplemented with HAT (hypoxanthine, aminopterin, and thymidine). As selection and culture media, any media can be used, provided that hybridomas can grow therein. The antibody titer in the hybridoma culture supernatant can be measured in the same manner as with the measurement of the antibody titer in the antiserum.

Monoclonal antibodies can be separated and purified by conventional techniques for separation and purification of polyclonal antibodies. Examples of such techniques include salting out, alcohol precipitation, isoelectric precipitation, electrophoresis, adsorption and/or desorption by ion exchangers (e.g., DEAE), ultracentrifugation, gel filtration, and separation and purification of immunoglobulin by a specific purification technique using an antigen-binding solid phase or a specific purification technique using protein A or G.

In contrast, polyclonal antibodies can be prepared, for example, in the manner described below.

Polyclonal antibodies can be prepared by, for example, constructing an antigen-carrier composite, immunizing a mammalian in the same manner as described with regard to the method for preparing monoclonal antibodies, extracting a substance containing an antibody reacting with BAng-25 from the immunized animal, and separating and purifying the antibodies. The antigens used for preparing monoclonal antibodies can be used for preparing polyclonal antibodies. When constructing an antigen-carrier composite, any carriers may be used, and antigens may be crosslinked to carriers in any amounts, provided that antibodies can be efficiently produced relative to the amount of the antigens crosslinked to the carriers. Examples of carriers that can be used include bovine serum albumin, bovine thyroglobulin, and keyhole limpet hemocyanin. Antigens can be coupled to carriers with the use of various types of condensation agents. Specific examples of condensation agents that can be used include glutaraldehyde, carbodiimide, and an active ester reagent containing maleimide active ester, a thiol group, and a dithiopyridyl group.

The antigen-carrier composite is administered to an animal to be immunized by itself or together with a carrier or a diluent at a site at which an antibody can be produced. In order to enhance the capacity for antibody production, complete Freund's adjuvant or incomplete Freund's adjuvant may also be administered. Administration can be generally carried out about once every 2 to 6 weeks up to about 3 to 10 instances in total. The same mammalians as those used for the preparation of monoclonal antibodies can be used. Polyclonal antibodies can be sampled from, for example, the blood and the ascites, and preferably from the blood of the animals immunized in the manner described above. The polyclonal antibody titer in the antiserum can be measured in the same manner as with the measurement of the antibody titer in the serum. Polyclonal antibodies can be separated and purified in the same manner as in the case of separation

and purification of the monoclonal antibodies described above.

The antibody of the present invention may be adequately labeled for the purpose of detection. Examples of label substances that can be used include enzymes, radioisotopes, and fluorescent dyes. Alternatively, a substance that binds specifically to the antibody of interest, such as protein A or protein G, may be labeled instead of the antibody, so as to indirectly detect the antibody of interest.

Immunological assay techniques for measuring the amount of BAng-25 involving the use of the antibody of the present invention are not particularly limited. For example, conventional techniques, such as enzyme immunoassay (EIA), latex agglutination assay, immunochromatography, Western blotting, radioimmunoassay (RIA), fluorescence immunoassay (FIA), luminescence immunoassay, spin immunoassay, turbidimetry comprising measuring the turbidity caused by antigen-antibody composite formation, the enzyme sensor electrode method comprising detecting potential changes caused by binding to an antigen with the use of an antibody solid phase membrane electrode, and immunoelectrophoresis, can be employed. EIA and Western blotting are particularly preferable. Also, competitive enzyme immunoassay, sandwich enzyme-linked immunosorbant assay (sandwich ELISA), and other techniques are within the scope of the EIA techniques.

In the case of sandwich ELISA, for example, an antibody that binds specifically to BAng-25 is allowed to bind to a solid phase, the biological sample obtained from the subject (a urine sample, preferably) is added thereto, the solid phase is washed, a secondary antibody reacting with human immunoglobulin labeled with an enzyme such as alkaline phosphatase or peroxidase is added and allowed to react therewith, a substrate (a color developer) selected in accordance with the enzyme is added, and the developed color is assayed by measuring the absorbance of the reaction solution using a spectrophotometer or fluorometer. Thus, BAng-25 in the biological sample can be quantified. Examples of solid phases that can be used include polyvinyl chloride, polystyrene, polyethylene, polypropylene, polyester, fluorocarbon resin, glass, and metal. Carriers may be in the form of, for example, plates, beads, cells, or test tubes. It is preferable that a commercially available solid phase, such as a microtiter plate for enzyme immunoassay (e.g., a 96-well microtiter plate), be used.

When a renal disease or a cardiovascular disease of a subject is diagnosed on the basis of the amount of BAng-25 measured in the manner described above, it is preferable to compare the measured BAng-25 value with the reference value. When a subject is evaluated regarding whether or not he/she is afflicted with a disease of interest, for example, the amount of BAng-25 in the biological sample obtained from a healthy individual can be employed as the reference value. When evaluating therapeutic effects, the amount of BAng-25 in the biological sample obtained from the subject before treatment can be employed as the reference value. In the present invention, such specimens to be compared are referred to as "control samples."

When diagnosis is made in comparison with a control sample, it is determined that the subject has a renal disease or a cardiovascular disease, or it is determined that conditions of the renal disease or the cardiovascular disease of the subject are more serious than those associated with the control sample if the amount of BAng-25 in the biological sample obtained from the subject is greater than that of the control sample to a significant extent.

When a diagnosis is made regarding whether or not a subject is afflicted with a renal disease or a cardiovascular disease using a urine sample, more typically, the amount of BAng-25 in the urine sample obtained from the subject is measured, and the measured amount is compared with the amount of BAng-25 in a urine sample obtained from a control healthy individual. If the former value is significantly higher than the latter value, it is highly likely that the subject is afflicted with the renal disease or the cardiovascular disease. When evaluating therapeutic effects on the renal disease or the cardiovascular disease, a urine sample obtained from the subject before the treatment is used as a control sample. If the amount of BAng-25 in the urine sample obtained from the subject after the treatment is smaller than that of the control sample (i.e., before the treatment) to a significant extent, therapeutic effects can be verified. In such a case, the amount of BAng-25 in the control urine sample can be measured in the same manner as in the case of measurement of the amount of BAng-25 in the urine sample obtained from the subject. The amount of BAng-25 in the control urine sample may be measured every time the urine sample of the subject is analyzed, or it may be measured in advance. At the time of diagnosis or evaluation, the ratio of the BAng-25 level to the urine creatinine level in the urine sample may be employed as the indicator. When a blood sample is employed as a biological sample, also, diagnosis or evaluation may be carried out in the same manner as in a case in which a urine sample is used.

3. Method for screening for therapeutic agent for diabetic nephropathy or cardiovascular disease

The present invention also relates to a method for screening for a therapeutic agent for a diabetic nephropathy or a cardiovascular disease.

The screening method of the present invention comprises bringing a test substance into contact with mammalian-derived glomerular epithelial cells or administering a test substance to a non-human mammalian and measuring the amount of BAng-25 in the cell, urine, or blood sample, and investigating changes in such amount. If the test substance decreases the amount of BAng-25, such test substance can be a candidate for a therapeutic agent for a diabetic

nephropathy or a cardiovascular disease.

Test substances are preferably brought into contact with, in addition to glomerular epithelial cells, cells derived from the kidneys, the heart, or the blood vessels, such as cardiac muscle cells, vascular endothelial cells, smooth muscle cells, mesangium cells, proximal tubular epithelial cells, fibroblasts, immunocytes, or interstitial cells. In addition, cells or adipocytes derived from angiotensinogen-producing organs, such as the brain, the adrenal gland, or the ovary, may be used. Cells derived from either humans or non-human mammalians may be used.

When the test substance is administered to non-human mammalian animals, use of animal models of nephropathy or hypertension, such as podocyte injury-induced mouse models (NEP25 mice), HIGA mice, spontaneously hypertensive rats (SHR), Dahl salt-sensitive rats, stroke-prone spontaneously hypertensive rats (SHRSP), Tukuba hypertensive mice, or hereditary nephrotic ICGN mice, is preferable.

Target test substances of the screening method of the present invention are not particularly limited. Examples of target test substances include: a mixture containing a plurality of compounds, such as animal and/or plant tissue extracts or cultured microorganisms, or purified specimens thereof; naturally occurring molecules (e.g., amino acids, peptides, oligopeptides, polypeptides, proteins, nucleic acids, lipids, steroids, glycoproteins, or proteoglycans); synthesized analogues or derivatives of naturally occurring molecules (e.g., peptide mimics); non-naturally occurring molecules (e.g., low-molecular-weight organic compounds prepared via combinatorial chemistry); and mixtures thereof. A single substance may be independently tested, or a mixture of several candidate substances (including a library) may be tested. Examples of libraries including a plurality of test substances include synthetic compound libraries and peptide libraries.

4. Agent and kit for diagnosis of diabetic nephropathy or cardiovascular disease

The present invention also relates to an agent for diagnosis of a diabetic nephropathy or cardiovascular disease comprising an antibody that binds specifically to BAng-25.

The agent for diagnosis of the present invention can be prepared in the form of a kit that includes a necessary reagent. For example, such kit can include necessary constituents, such as an antibody that binds specifically to BAng-25, a labeled secondary antibody, and a reagent for detection. The kit may further include a standard sample, a buffer, a solution, a washing liquid, a reaction terminator, and instructions.

Antibodies that bind specifically to BAng-25 may be applied to a chip at high density to prepare a protein chip, and such protein chip may be included in the agent for diagnosis of the present invention.

Hereafter, the present invention is described in greater detail with reference to the examples, although the technical scope of the present invention is not limited to these examples.

[Example 1]

1. Method of experimentation

(1) Method of purification

Urine samples (5.5 liters) were obtained from 3 healthy individuals. Subsequently, the urine samples were applied to a Sep-Pak C18 cartridge and eluted with the aid of 0.1% trifluoroacetic acid (TFA)-containing 60% acetonitrile. The eluted sample was applied to a CM 52 column eluted with 0.1M $NH_4COOH$ (pH 4.0) and then subjected to gel filtration using Sephadex C-50. The peptide extract was subjected to reversed-phase high-performance liquid chromatography (RP-HPLC) using a diphenyl column and affinity chromatography using an affinity column prepared with the anti-N-terminal Ang II antiserum (Affi-Gel 10 Active Ester Agarose; Bio Rad). At the end, the purified peptide was subjected to RP-HPLC using an ODS-120A column (elution gradient: a 10% to 60% linear gradient of acetonitrile dissolved in 0.1% trifluoroacetic acid at a flow rate of 1 ml/min for 60 minutes).

The purification process described above was performed via radioimmunoassay (RIA) while monitoring immunoreactive N-terminal Ang II in accordance with the literature (S. Nagata, et al., Isolation and identification of proangiotensin-12, a possible component of the renin-angiotensin system, Biochem. Biophys. Res. Commun., 350, 2006, 1026-1031).

(2) Determination of structure

In order to determine the amino acid sequence and the molecular weight of the purified peptide, tandem mass spectrometry was carried out via positive electrospray ionization using the QSTAR Elite Hybrid LC/MS/MS System (AB SCIEX, U.S.A.) and the Procise 494 HT Protein Sequencing System (Applied Biosystems, U.S.A.). The sample was dissolved in a 0.1% formic acid-containing 50% acetonitrile solution. The conditions for enzymatic removal for glycosyl chain determination, fluorescent labeling, and separation and structural identification of N-glycan were in accordance

with the literature (H. Nakagawa, et al., Identification of neutral and sialyl N-linked oligosaccharide structures from human serum glycoproteins using three kinds of high-performance liquid chromatography, Anal. Biochem., 226, 1995, 130-138; H. Yagi, et al., Development of structural analysis of sulfated N-glycans by multidimensional high performance liquid chromatography mapping methods, Glycobiology 15, 2005, 1051-1060). Specifically, the defatted extract was subjected to protein decomposition using pepsin and glycoamidase A, and the resulting peptide was further treated with pronase and converted into an amino acid. After purification was carried out via Bio-Gel P-2 chromatography, the reducing terminus of the removed N-glycan was labeled with fluorescent 2-aminopyridine. After an excess reagent was removed via Sephadex G-15 chromatography, pyrimidylamino (PA)-glycan was separated on the basis of the charge using a TSK-gel diethylaminoethyl (DEAE)-5PW column (Tosoh Corporation, Tokyo, Japan). The separated fractions were applied to a Shim-pack HRC octadecyl silica (ODS) column (Shimadzu Corporation, Kyoto, Japan) for further separation on the basis of hydrophobicity, and the elution time at each peak was represented in terms of the glucose unit (GU) value. The molecular weight of a PA-glycan fraction was analyzed via MALDI-TOF-MS. A fraction containing two types of glycans was further subfractionated in accordance with the molecular weight using a TSK-gel Amide-80 column (Tosoh), and the position of elution was determined on the basis of the GU value. The PA glycan samples were subjected to mapping based on the GU values and molecular weights thereof, and the coordinates thereof were compared with those of reference PA-glycans in the GALAXY database.

(3) Conversion of big angiotensin-25

(3-1) Examination of renin reactivity

Three types of peptides were synthesized: big angiotensin-25 equivalent to the peptide, which is determined in terms of the structure in (2) (BAng-25); another big angiotensin-25 derived from the former big angiotensin-25 by deletion of the N-linked glycosyl chain (Non-glycosyl chain BAng-25); and another big angiotensin-25 derived from the former big angiotensin-25 by substitution of the N-linked glycosyl chain with GlucNAc (BAng-25+GlcNAc).

The three types of peptides (10 μg each) were subjected to incubation with recombinant human renin (100 U, AnaSpec, Inc., U.S.A.) *in vitro.* Incubation was carried out in 200 μl of 50 mM Tris-HCl (pH 8.0) containing 100 mM NaCl at 37°C for 1 hour. A half (5 μg) of each reaction product was subjected to gradient RP-HPLC described above.

(3-2) Examination of chymase reactivity

The three types of peptides (10 μg each) were subjected to incubation with 43.4 mU of human skin mast-cell-derived chymase (Elastin Products Company, Inc., U.S.A.) *in vitro.* Incubation was carried out in 200 μl of 600 mM Tris-HCl (pH 8.0) containing 3 M NaCl at 25°C for 1 hour. A half (5 μg) of each reaction product was subjected to RP-HPLC.

- Conditions:

Column: ODS

Buffer A: Water:acetonitrile:10%TAF = 90:10:1
Buffer B: Water:acetonitrile:10%TAF = 40:60:1

A gradient of 100% Buffer A to 100% Buffer B over a period of 1 hour

(4) Immunostaining of human big angiotensin-25

In order to specifically detect human big angiotensin-25, the antiserum against the C-terminus of the peptide was used. An immunostaining agent using an antibody was prepared using big angiotensin-25 (18-25) (Scrum Inc., Tokyo, Japan). According to the conventional technique, a formalin-fixed and paraffin-embedded tissue block was cut into 4-μm sections and immunohistochemicallly stained with the use of an anti-rabbit polyclonal antibody (diluted 100-fold with PBS). Non-immune rabbit serum (Dako Japan Inc.) was used as a negative control sample. The sections were stained with EnVision+ (Dako Japan Inc.). Horseradish peroxidase activity was visualized using 3,3-diaminobenzidine containing hydrogen peroxide, and the sections were subjected to contrast staining using Mayer's hematoxylin.

(5) Collection of urine sample and measurement of big angiotensin-25

Subjects (518 individuals) were recruited at medical institutions in Miyazaki City, Miyazaki Prefecture, Japan. The urine samples were applied to Sep-Pak C18 cartridges, elution was carried out with 60% acetonitrile containing

0.1% trifluoroacetic acid (TFA), and immunoreactive N-terminal big angiotensin-25 of each sample was measured via RIA. Proteinuria, urine creatinine, and serum creatinine were assayed at SRL, Inc. (Tokyo, Japan). The estimated Glomerular Filtration Rate (eGFR) was calculated using the following equation.

$$\mathrm{eGFR\ (ml/min/1.73\ m^2)} = 194 \times \mathrm{age}^{-0.287} \times \mathrm{serum\ creatinine\ (mg/dl)}^{-1.094}$$

(for female: x 0.739)

(6) Statistical analysis

The unpaired t-test was carried out to compare the data of the proteinuria-positive (+) group and that of the proteinuria-negative (-) group. The eGFR data were compared via variance analysis (ANOVA), followed by Bonferroni's test. The numerical values each indicate "mean ± S.E." The statistical significance was determined to be P < 0.05.

2. Results

(1) Structural analysis of big angiotensin-25

Human urine was subjected to gel filtration, immunoreactivity of each fraction was measured via radioimmunoassay (RIA) specific to the angiotensinogen N-terminus, and a fraction with high activity was found at a molecular weight of about 5,000 (Fig. 1A). More specifically, this fraction was found to contain the most critical peptide among the peptides associated with angiotensin in urine. Subsequently, the fraction having high immunoreactivity was repeatedly subjected to purification through ion exchange chromatography, affinity purification, and reversed-phase high-performance liquid chromatography (RP-HPLC) (elution gradient: a linear elution gradient of 10% to 60% acetonitrile dissolved in 0.1% trifluoroacetic acid at a flow rate of 1 ml/min for 60 minutes), and the single peak was observed at the end (Fig. 1B).

The purified peptide sample was subjected to mass spectrometry, and the peaks of hexavalent ions (780.2 m/z), pentavalent ions (936.0 m/z), and quadrivalent ions (1169.8 m/z) were detected (Fig. 1C). On the basis thereof, the molecular weight was found to be 4675.1; however, this did not lead to structural analysis, and a peak indicating glycosyl chain addition was detected. Further, the glycosyl chain of the purified peptide was cleaved with N-glucosidase and subjected to mass spectrometry. As a result, pentavalent ions (611.71 m/z) and quadrivalent ions (764.39 m/z) were detected (data not shown).

The amino acid sequence of the purified peptide was analyzed using a protein sequencer. As a result, 25 amino acids were detected, and the dehydro-form of Cys was detected at position 18. This sequence was found to be equivalent to the N-terminus of angiotensinogen, the amino acid at position 14 was found to be Asn, and the presence of a glycosyl chain was confirmed via mass spectrometry. Thus, an N-linked glycan was considered to have been added to an amino acid at position 14 (Fig. 1D). As a result of mass spectrometry, also, the amino acid at position 18 was found to be Cys-Cys (cystine) (Fig. 1D).

As a result of primary analysis of the N-linked glycan contained in the purified peptide, a single type of neutral sugar (molecular weight: 1,720 Da; sugar composition: (Hex)5(HexNAc)4(PA)1)) was detected (Fig. 2A). This N-linked glycan was further fractionated using an amide column, and the glycosyl chain structure was identified via mapping on the basis of the HPLC elution position (elution position of the ODS column: 9.8; elution position of the amide column: 6.9) and the results of MALDI-TOF-MS. As a result, the coordinate of the N-linked glycan was found to be identical to that of the known reference N-linked glycan in GALAXY (code no. 200.4). This candidate glycosyl chain and the purified glycosyl chain were subjected to co-chromatography, and the peaks thereof were found to be identical to each other (Fig. 2B).

The purified peptide was designated "Big angiotensin-25." Big angiotensin-25 has a characteristic structure such that it comprises 25 amino acids (SEQ ID NO: 1), an N-linked glycan added thereto at position 14, and cysteine further added thereto at position 18 (Fig. 2C).

(2) Conversion of big angiotensin-25 into angiotensin I (Ang I) and angiotensin II (Ang II)

(2-1) Renin reactivity

Fig. 4 shows the results of incubation of the three types of peptides (Fig. 3); i.e., big angiotensin-25 with a glycosyl chain (BAng-25), big angiotensin-25 without a glycosyl chain (Non-glycosyl chain BAng-25), and big angiotensin-25 with GlcNAc substitution (BAng-25+GlcNAc), with renin. While BAng-25 was not cleaved by renin and Ang I was not

produced (Fig. 4B), Non-glycosyl chain BAng-25 (Fig. 4D) and BigAng-25+GlcNAc (Fig. 4F) were cleaved by renin, and Ang I was produced.

Fig. 5 shows the results of reactions of big angiotensin-25 without a glycosyl chain (Non-glycosyl chain BAng-25) and big angiotensin-25 with a glycosyl chain (BAng-25) in the presence of 20 U of renin for 24 hours. While Ang I was immediately produced in the absence of a glycosyl chain, Ang I was not produced after the elapse of 24 hours in the presence of a glycosyl chain. The results demonstrate that BAng-25 with a glycosyl chain is resistant against renin. Thus, the presence or absence of a s glycosyl chain was considered to be deeply related to the enzyme specificity of renin.

(2-2) Chymase reactivity

Fig. 6 shows the results of incubation of the three types of peptides; i.e., big angiotensin-25 with a glycosyl chain (BAng-25), big angiotensin-25 without a glycosyl chain (Non-glycosyl chain BAng-25), and big angiotensin-25 with GlcNAc substitution (BAng-25+GlcNAc), with chymase. While BAng-25 (Fig. 6B) and Non-glycosyl chain BAng-25 (Fig. 6E) were cleaved by chymase and Ang II was produced, BAng-25+GlcNAc (Fig. 6F) was not cleaved by chymase and Ang II was not produced. The fact that Ang II was produced as a result of the reaction between BAng-25 and chymase indicates the presence of the renin-independent Ang II production pathway.

Fig. 7 shows the results of reactions of the three types of peptides (10 μg each); i.e., big angiotensin-25 with a glycosyl chain (BAng-25), big angiotensin-25 without a glycosyl chain (Non-glycosyl chain BAng-25), and big angiotensin-25 with GlcNAc substitution (BAng-25+GlcNAc), with 0.4 μl of chymase in 190 μl of a buffer (0.6 M Tris-HCl (pH 8.0), 0.3 M NaCl) at 25 °C for 24 hours. While BAng-25 and Non-glycosyl chain BAng-25 immediately produced Ang II, BAng-25+GlcNAc had not produced Ang II after the elapse of 24 hours.

The reactivity of big angiotensin-25 with enzyme is summarized as follows.

Big angiotensin-25 is more resistant against renin than angiotensinogen or BAng-25 peptide without an N-linked glycan, although it is reactive with chymase. In the absence of a glycosyl chain, big angiotensin-25 is reactive with both renin and chymase. Big angiotensin-25 with GlcNAc substitution is reactive with renin, although it is not reactive with chymase.

Accordingly, presence or absence and type of glycosyl chain were found to influence the effects of renin and chymase.

(3) Expression of human big angiotensin-25 in kidneys

The results of tissue staining were visualized in the kidneys of the surgical samples, and glomerular epithelial cells (podocytes) were found to be specifically stained (Fig. 8A).

(4) Correlation with urine big angiotensin-25

Fig. 8B shows the urine big angiotensin-25/creatinine ratios of the three groups (eGFR (ml/min/1.73m$^2$): more than 90; 90 to 30; and less than 30) in urine. The urine big angiotensin-25/creatinine ratio of the group with eGFR of less than 30 (severe nephropathy) was significantly higher than that of other two groups. When proteinuria-positive cases (Upro(+)) were compared with proteinuria-negative cases (Upro(-)) among diabetic patients, also, the urine big angiotensin-25/creatinine ratio was significantly higher in the case of proteinuria-positive cases (Upro(+)), compared with negative cases (Upro(-)) (Fig. 8C). In addition, the urine big angiotensin-25/creatinine ratio was found to be positively correlated with proteinuria (creatinine correction) (Fig. 8D).

(5) Expression of human big angiotensin-25 in other tissue

The results of immunostaining of the tissue blocks of organs prepared from surgical samples (e.g., the heart, the esophagus, the adrenal gland, the pancreas, the stomach, the large intestine, the placenta, the salpinx, and the testicle) were visualized (Figs. 9 to 17). As a result, intensely stained cells were observed in cardiac muscle cells, islets of Langerhans, adrenal medulla, Sertoli cells of the testicle, smooth muscle cells (SMC) of the salpinx, and extravillous trophoblast cells in the placenta (Table 1).

Table 1

| Organs | Tissues | Expression |
|---|---|---|
| Kidneys | Glomerular epithelial cells | ++ |
| Heart | Left ventricle, cardiac muscle cells | ++ |

(continued)

| Organs | Tissues | Expression |
|---|---|---|
| Lung | Pulmonary artery, SMC (smooth muscle cells)<br>Bronchiole, epithelium | +<br>+ |
| Esophagus | Stratified squamous epithelium (basal layer) | + |
| Stomach | Glands (endocrine cells) Nerve plexus (submucosal or Auerbach's plexus) | ++ |
| Large intestine | Crypt cells (endocrine cells) | + |
| Liver | Hepatic cells | + |
| Spleen | Histocvtes | + |
| Pancreas | Pancreatic islets (islets of Langerhans)<br>Exocrine glands (acinus/ducts) | ++<br>- |
| Adrenal gland | Medulla<br>Cortex | ++<br>- |
| Lymph node | Histocytes<br>Dendritic cells | +<br>+ |
| Testicle | Sertoli cells | ++ |
| Salpinx | SMC (smooth muscle cells)<br>Tubal epithelium | ++<br>+ |
| Placenta | Extravillous trophoblast cells<br>Villus | ++<br>- |

Industrial Applicability

The present invention can be utilized in the field of production of an agent or kit for diagnosis of a renal disease or a cardiovascular disease.

## Claims

1. A peptide consisting of the amino acid sequence as shown in SEQ ID NO: 1 which has a disulfide bond involving the cysteine residue at position 18 of the amino acid sequence as shown in SEQ ID NO: 1 and another cysteine or an amino acid sequence derived therefrom by deletion, substitution, or addition of 1 to 5 amino acids other than the cysteine residue at position 18 and other than the asparagine residue at position 14 and an N-linked glycan added thereto at the asparagine residue at position 14, wherein the peptide is resistant against renin and reactive with chymase.

2. The peptide according to claim 1, wherein the N-linked glycan may optionally comprise sialic acid bound thereto at the non-reducing terminus and the glycosyl chain is constituted by sugars, including galactose, N-acetylglucosamine, and mannose.

3. The peptide according to claim 1 or 2, wherein the N-linked glycan has a structure represented by Formula (I), (II), or (III):

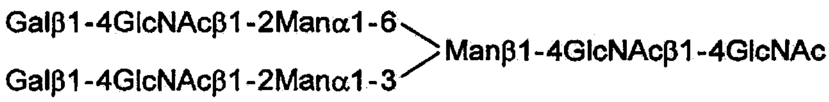
(I)

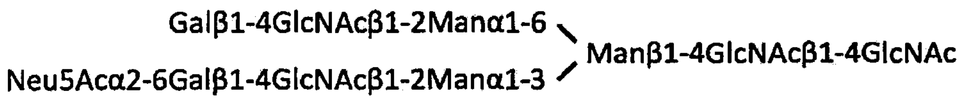
(II)

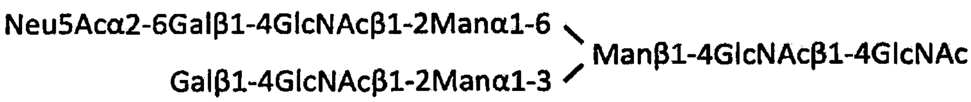
(III)

wherein, in the above Formula (I), (II), or (III), Gal represents galactose; Man represents mannose; GlcNAc represents N-acetylglucosamine; and Neu5Ac represents N-acetylneuraminic acid.

4. Use of the peptide according to any of claims 1 to 3 as a diagnostic marker for diagnosing a diabetic nephropathy or a cardiovascular disease in vitro.

5. In vitro use of the peptide according to any of claims 1 to 3 as a chymase substrate.

6. A test method for diagnosis of diabetic nephropathy or a cardiovascular disease comprising measuring the amount of the peptide according to any of claims 1 to 3 in a biological sample that has been obtained from a subject.

7. A method for evaluating effects of treating a diabetic nephropathy or a cardiovascular disease comprising investigating a change in the amount of the peptide according to any of claims 1 to 3 in biological samples that have been obtained from a subject before and after the treatment.

8. The method according to claim 6 or 7, wherein the biological sample is urine.

9. A method for screening for a therapeutic agent for a diabetic nephropathy or a cardiovascular disease comprising bringing a test substance in vitro into contact with mammalian-derived glomerular epithelial cells or administering a test substance to a non-human mammalian and measuring the amount of the peptide according to any of claims 1 to 3 in the cells or urine to investigate a change in the amount of the peptide.

10. The method according to any of claims 6 to 9, wherein the amount of the peptide is measured via immunological assay using an antibody that binds specifically to a peptide consisting of residues 18 to 25 of the amino acid sequence as shown in SEQ ID NO:1.

11. An antibody that binds specifically to a peptide consisting of residues 18 to 25 of the amino acid sequence as shown in SEQ ID NO:1.

12. An agent for diagnosis of a diabetic nephropathy or a cardiovascular disease comprising the antibody that binds specifically to a peptide consisting of residues 18 to 25 of the amino acid sequence as shown in SEQ ID NO:1.

13. A kit for diagnosis of a diabetic nephropathy or a cardiovascular disease comprising the antibody that binds specifically to a peptide consisting of residues 18 to 25 of the amino acid sequence as shown in SEQ ID NO:1.

## Patentansprüche

1. Peptid, das aus der in SEQ ID NO:1 gezeigten Aminosäuresequenz besteht, das eine Disulfidbindung aufweist, die den Cysteinrest an Position 18 der wie in SEQ ID NO:1 gezeigten Aminosäuresequenz und ein weiteres Cystein umfasst, oder aus einer Aminosäuresequenz, die davon durch Deletion, Substitution oder Hinzufügung von 1 bis 5 Aminosäuren außer dem Cysteinrest an Position 18 und außer dem Asparaginrest an Position 14 abgeleitet ist, und einem N-gebundenen Glycan, das davon am Asparaginrest an Position 14 hinzugefügt ist, wobei das Peptid gegen Renin resistent ist und mit Chymase reaktiv ist.

2. Peptid nach Anspruch 1, wobei das N-gebundene Glycan gegebenenfalls Sialinsäure umfasst, die daran am nicht-reduzierenden Terminus gebunden ist, und wobei die Glycosylkette aus Zuckern einschließlich Galactose, N-Acetylglucosamin und Mannose besteht.

3. Peptid nach Anspruch 1 oder 2, wobei das N-gebundene Glycan eine Struktur aufweist, die durch Formel (I), (II) oder (III) dargestellt ist:

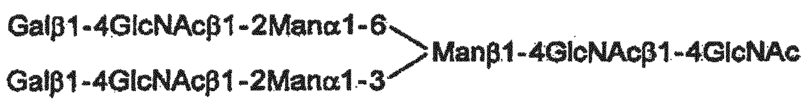
(I)

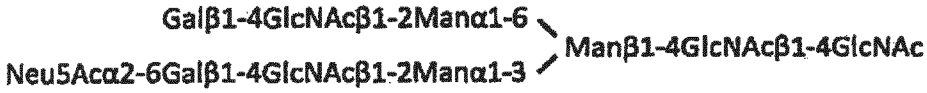
(II)

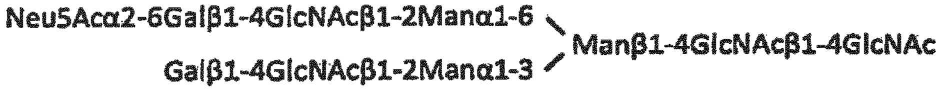
(III)

,

wobei in der obigen Formel (I), (II) oder (III), Gal Galactose darstellt; Man Mannose darstellt; GlcNAc N-Acetylglucosamin darstellt; und Neu5Ac N-Acetylneuraminsäure darstellt.

**4.** Verwendung des Peptids nach einem der Ansprüche 1 bis 3 als Diagnosemarker zum Diagnostizieren einer diabetischen Nephropathie oder einer kardiovaskulären Erkrankung *in vitro.*

**5.** *In vitro*-Verwendung des Peptids nach einem der Ansprüche 1 bis 3 als Chymasesubstrat.

**6.** Testverfahren zur Diagnose von diabetischer Nephropathie oder einer kardiovaskulären Erkrankung, umfassend das Messen der Menge des Peptids nach einem der Ansprüche 1 bis 3 in einer biologischen Probe, die von einem Individuum erhalten wurde.

**7.** Verfahren zum Beurteilen der Wirkung der Behandlung einer diabetischen Nephropathie oder einer kardiovaskulären Erkrankung, umfassend das Ermitteln einer Veränderung in der Menge des Peptids nach einem der Ansprüche 1 bis 3 in biologischen Proben, die von einem Individuum vor und nach der Behandlung erhalten wurden.

**8.** Verfahren nach Anspruch 6 oder 7, wobei die biologische Probe Urin ist.

**9.** Verfahren zum Screenen auf ein therapeutisches Agens für eine diabetische Nephropathie oder eine kardiovaskuläre Erkrankung, umfassend das Inkontaktbringen einer Testsubstanz mit von einem Säuger stammenden glomerulären Epithelzellen *in vitro* oder das Verabreichen einer Testsubstanz an einen nicht-humanen Säuger und das Messen der Menge des Peptids nach einem der Ansprüche 1 bis 3 in den Zellen oder dem Urin, um eine Veränderung in der Menge des Peptids zu ermitteln.

**10.** Verfahren nach einem der Ansprüche 6 bis 9, wobei die Menge des Peptids mittels immunologischem Assay unter Verwendung eines Antikörpers gemessen wird, der spezifisch an ein Peptid bindet, das aus den Resten 18 bis 25 der wie in SEQ ID NO:1 gezeigten Aminosäuresequenz besteht.

**11.** Antikörper, der spezifisch an ein Peptid bindet, das aus den Resten 18 bis 25 der wie in SEQ ID NO:1 gezeigten Aminosäuresequenz besteht.

**12.** Agens zur Diagnose einer diabetischen Nephropathie oder einer kardiovaskulären Erkrankung, umfassend den Antikörper, der spezifisch an ein Peptid bindet, das aus den Resten 18 bis 25 der wie in SEQ ID NO:1 gezeigten Aminosäuresequenz besteht.

**13.** Kit zur Diagnose einer diabetischen Nephropathie oder einer kardiovaskulären Erkrankung, umfassend den Antikörper, der spezifisch an ein Peptid bindet, das aus den Resten 18 bis 25 der in SEQ ID NO:1 gezeigten Aminosäuresequenz besteht.

## Revendications

**1.** Peptide constitué par la séquence d'acides aminés représentée dans SEQ ID No : 1 qui comporte une liaison disulfure impliquant le résidu cystéine à la position 18 de la séquence d'acides aminés représentée dans SEQ ID No : 1 et une autre cystéine ou une séquence d'acides aminés dérivée de celle-ci par délétion, substitution, ou addition de 1 à 5 acides aminés autres que le résidu cystéine à la position 18 et autres que le résidu asparagine à la position 14 et un glycane N-lié ajouté au niveau du résidu asparagine à la position 14, dans lequel le peptide résiste à la rénine et est réactif vis-à-vis de la chymase.

**2.** Peptide selon la revendication 1, dans lequel le glycane N-lié peut éventuellement comprendre un acide sialique lié à celui-ci à l'extrémité terminale non réductrice et la chaîne glycosyle est constituée de sucres, dont le galactose, la N-acétylglucosamine, et le mannose.

**3.** Peptide selon la revendication 1 ou 2, dans lequel le glycane N-lié a une structure représentée par la Formule (I), (II), ou (III) :

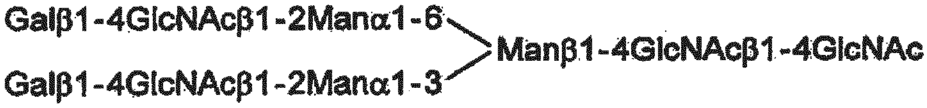
(I)

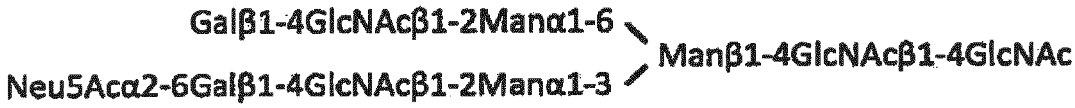
(II)

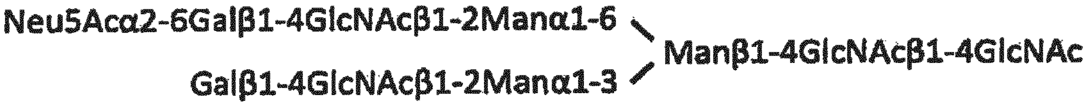
(III)

dans lequel, dans la Formule (I), (II), ou (III) ci-dessus, Gal représente le galactose ; Man représente le mannose ; GlcNAc représente la N-acétylglucosamine ; et Neu5Ac représente l'acide N-acétylneuraminique.

4. Utilisation du peptide selon l'une quelconque des revendications 1 à 3 comme marqueur diagnostique pour diagnostiquer une néphropathie diabétique ou une maladie cardiovasculaire in vitro.

5. Utilisation in vitro du peptide selon l'une quelconque des revendications 1 à 3 comme substrat de chymase.

6. Méthode d'essai pour le diagnostic d'une néphropathie diabétique ou d'une maladie cardiovasculaire comprenant la mesure de la quantité du peptide selon l'une quelconque des revendications 1 à 3 dans un échantillon biologique qui a été obtenu auprès d'un sujet.

7. Méthode pour évaluer les effets du traitement d'une néphropathie diabétique ou d'une maladie cardiovasculaire comprenant l'étude d'une variation de la quantité du peptide selon l'une quelconque des revendications 1 à 3 dans des échantillons biologiques qui ont été obtenus auprès d'un sujet avant et après le traitement.

8. Méthode selon la revendication 6 ou 7, dans laquelle l'échantillon biologique est l'urine.

9. Méthode de criblage pour identifier un agent thérapeutique pour traiter une néphropathie diabétique ou une maladie cardiovasculaire comprenant la mise en contact d'une substance d'essai in vitro avec des cellules épithéliales glomérulaires dérivées d'un mammifère ou l'administration d'une substance d'essai à un mammifère non humain et la mesure de la quantité du peptide selon l'une quelconque des revendications 1 à 3 dans les cellules ou l'urine pour étudier une variation de la quantité du peptide.

10. Méthode selon l'une quelconque des revendications 6 à 9, dans laquelle la quantité du peptide est mesurée par un dosage immunologique à l'aide d'un anticorps qui se lie spécifiquement à un peptide constitué par les résidus 18 à 25 de la séquence d'acides aminés représentée dans SEQ ID No : 1.

11. Anticorps qui se lie spécifiquement à un peptide constitué par les résidus 18 à 25 de la séquence d'acides aminés représentée dans SEQ ID No : 1.

12. Agent pour le diagnostic d'une néphropathie diabétique ou d'une maladie cardiovasculaire comprenant l'anticorps qui se lie spécifiquement à un peptide constitué par les résidus 18 à 25 de la séquence d'acides aminés représentée dans SEQ ID No : 1.

13. Kit pour le diagnostic d'une néphropathie diabétique ou d'une maladie cardiovasculaire comprenant l'anticorps qui se lie spécifiquement à un peptide constitué par les résidus 18 à 25 de la séquence d'acides aminés représentée dans SEQ ID No : 1.

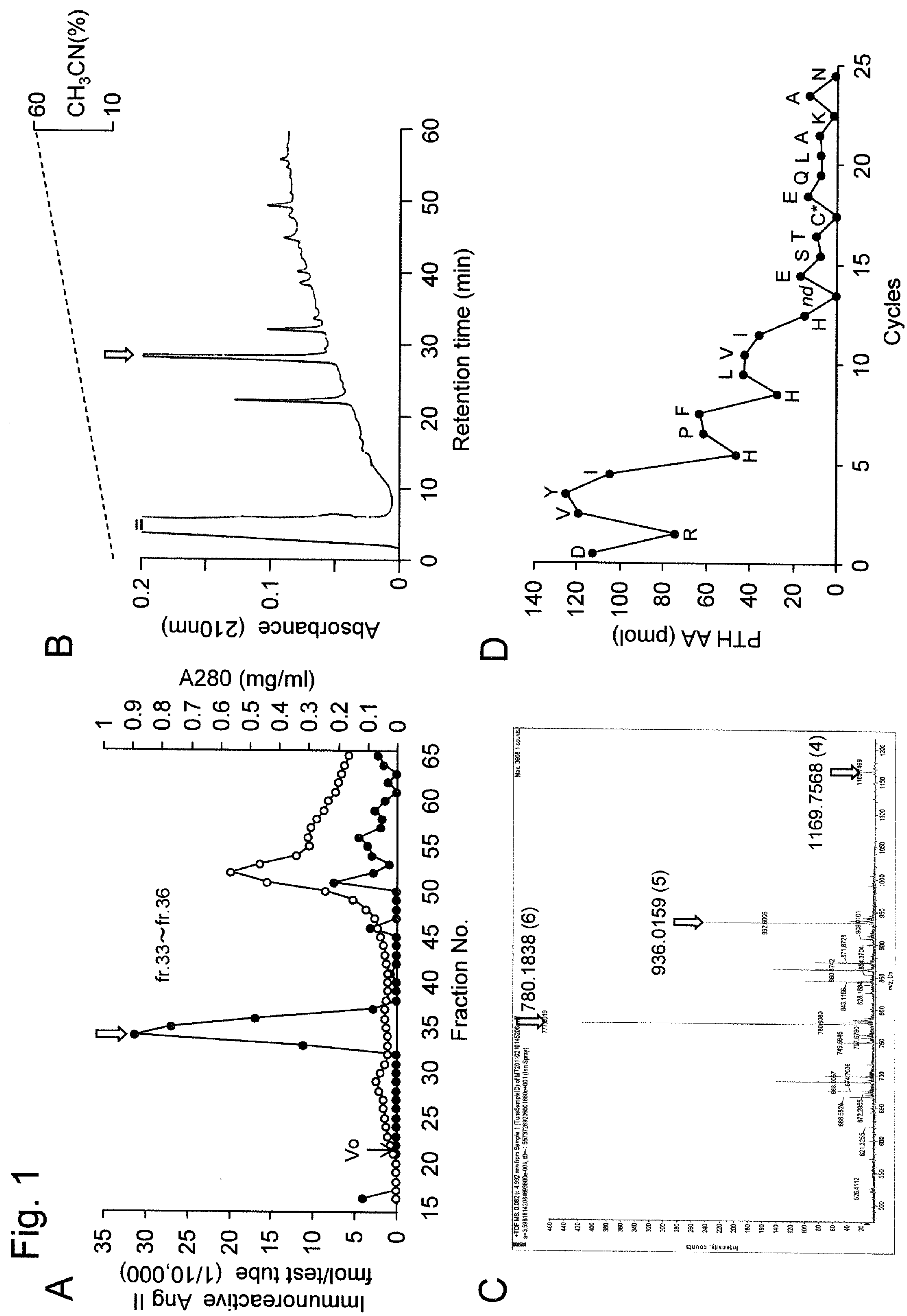
Fig. 1
A
Immunoreactive Ang II fmol/test tube (1/10,000)
A280 (mg/ml)
Fraction No.
Vo
fr.33~fr.36
B
Absorbance (210nm)
Retention time (min)
CH3CN(%)
II
C
780.1838 (6)
936.0159 (5)
1169.7568 (4)
D
PTH AA (pmol)
Cycles
D V Y I R H P F H L V I H nd E S T C* E Q L A K A N

Fig. 2

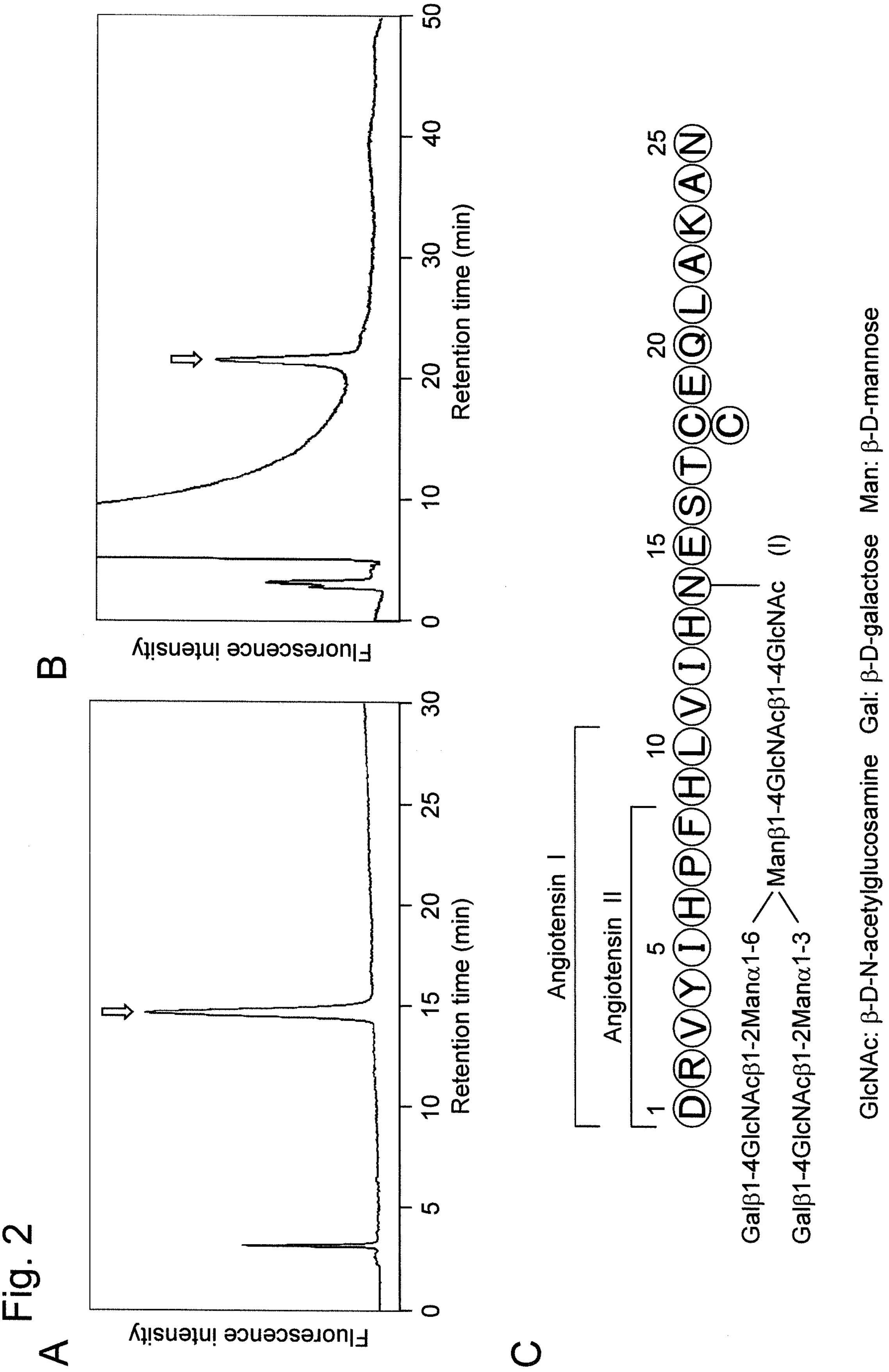
A
Fluorescence intensity
Retention time (min)
0
5
10
15
20
25
30
B
Fluorescence intensity
Retention time (min)
0
10
20
30
40
50
C
Angiotensin I
Angiotensin II
1
5
10
15
20
25
DRVYIHPFHLVIHNESTCEQLAKAN
C
Galβ1-4GlcNAcβ1-2Manα1-6
Galβ1-4GlcNAcβ1-2Manα1-3
Manβ1-4GlcNAcβ1-4GlcNAc
(I)
GlcNAc: β-D-N-acetylglucosamine Gal: β-D-galactose Man: β-D-mannose

Fig. 3

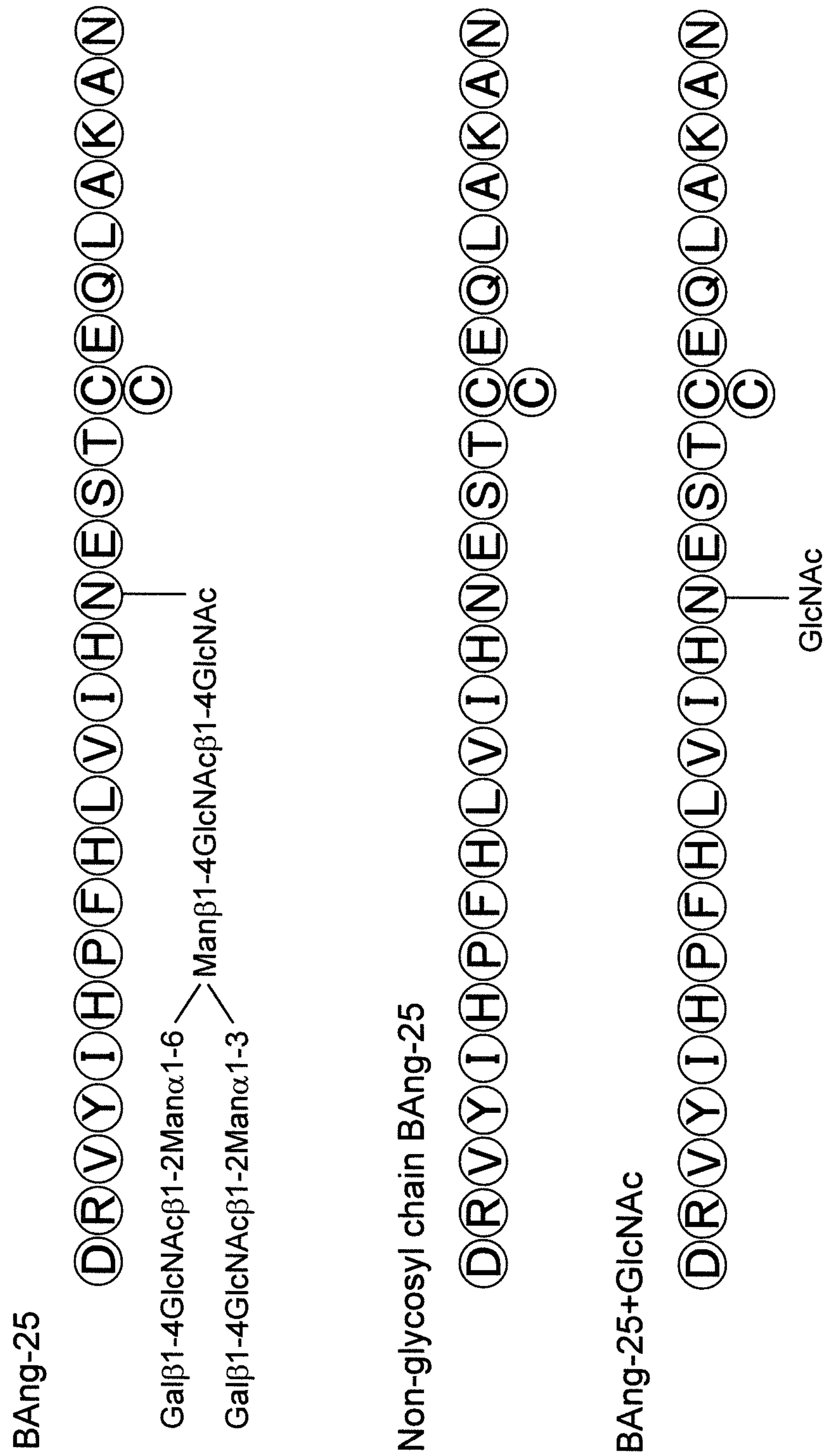
BAng-25
DRVYIHPFHLVIHNESTCEQLAKAN
C
Galβ1-4GlcNAcβ1-2Manα1-6
Galβ1-4GlcNAcβ1-2Manα1-3
Manβ1-4GlcNAcβ1-4GlcNAc
Non-glycosyl chain BAng-25
DRVYIHPFHLVIHNESTCEQLAKAN
C
BAng-25+GlcNAc
DRVYIHPFHLVIHNESTCEQLAKAN
C
GlcNAc

Fig. 4

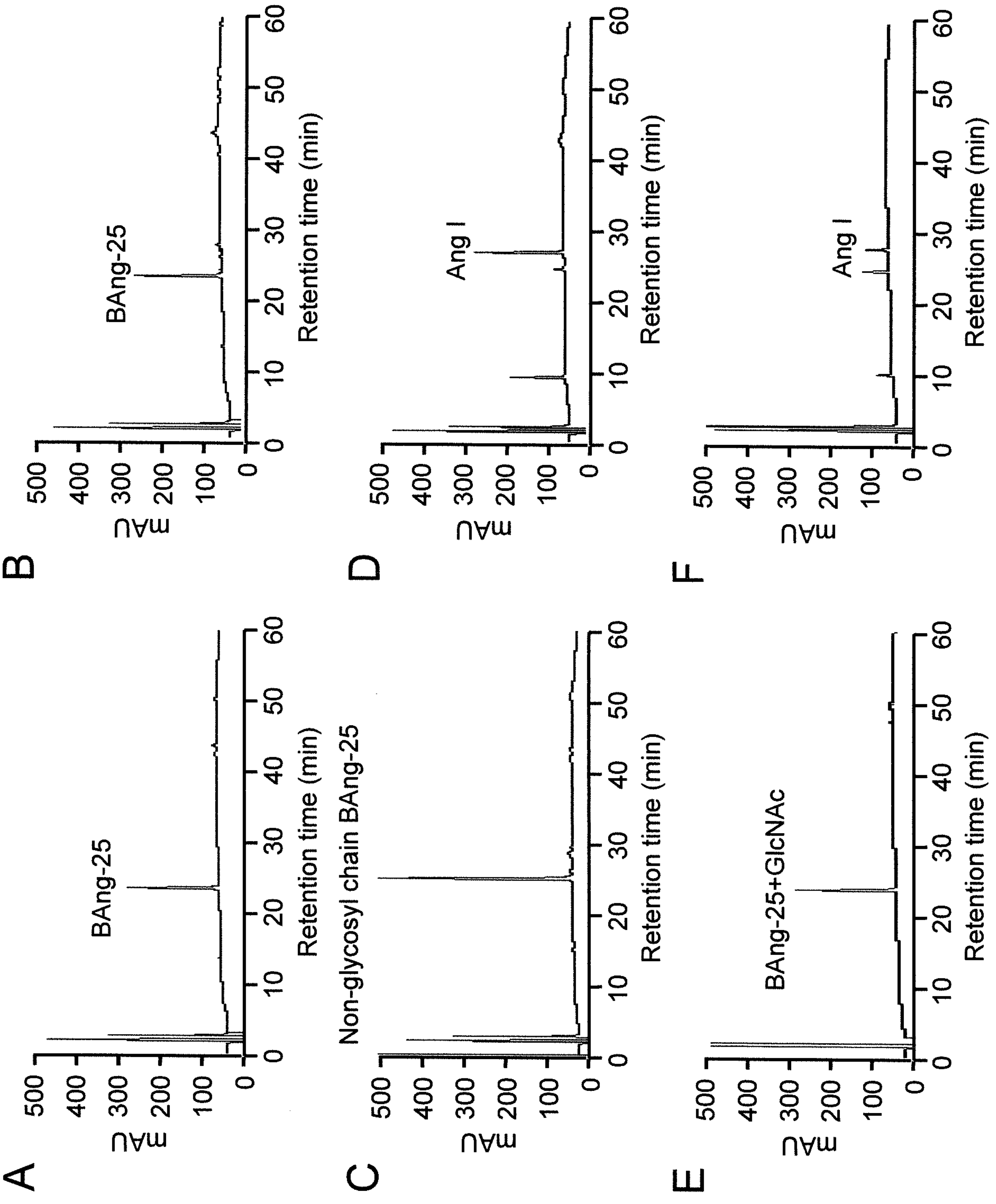
A
BAng-25
B
BAng-25
C
Non-glycosyl chain BAng-25
D
Ang I
E
BAng-25+GlcNAc
F
Ang I
mAU
Retention time (min)

Fig. 5

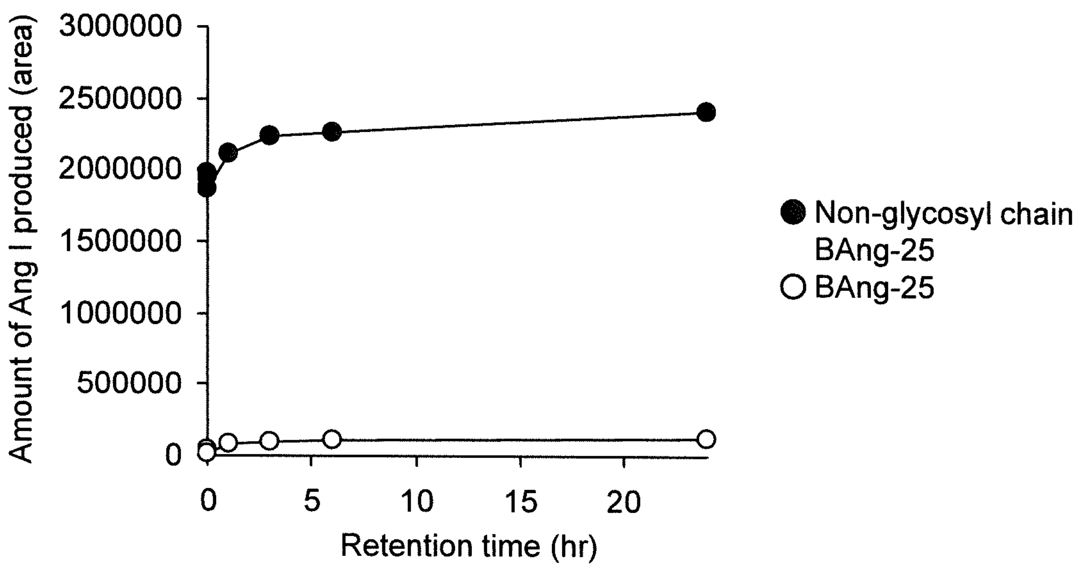
Amount of Ang I produced (area)
3000000
2500000
2000000
1500000
1000000
500000
0
0
5
10
15
20
Retention time (hr)
Non-glycosyl chain BAng-25
BAng-25

Fig. 6

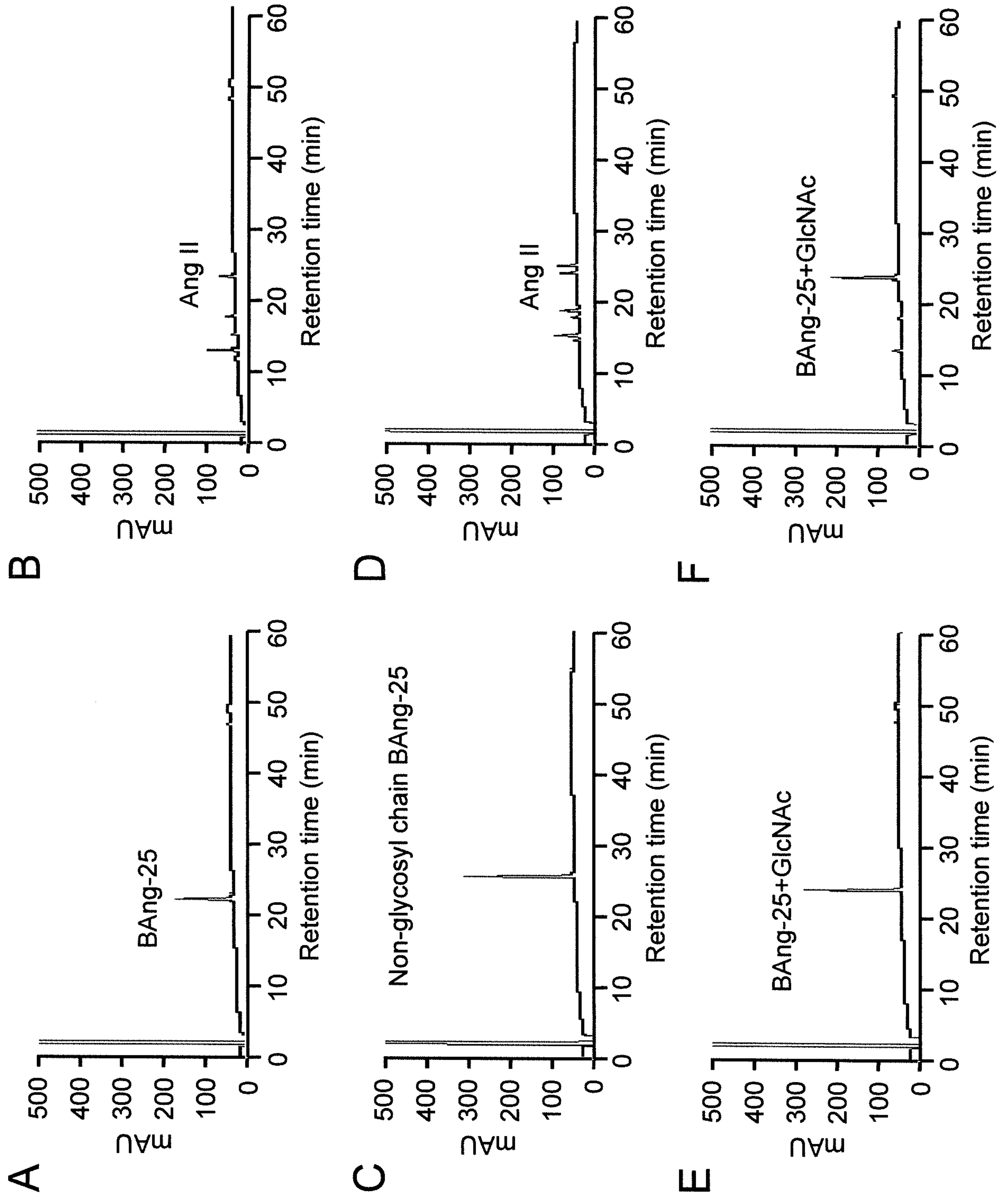
A
B
C
D
E
F
mAU
Retention time (min)
BAng-25
Ang II
Non-glycosyl chain BAng-25
Ang II
BAng-25+GlcNAc
BAng-25+GlcNAc
0
100
200
300
400
500
10
20
30
40
50
60

Fig. 7

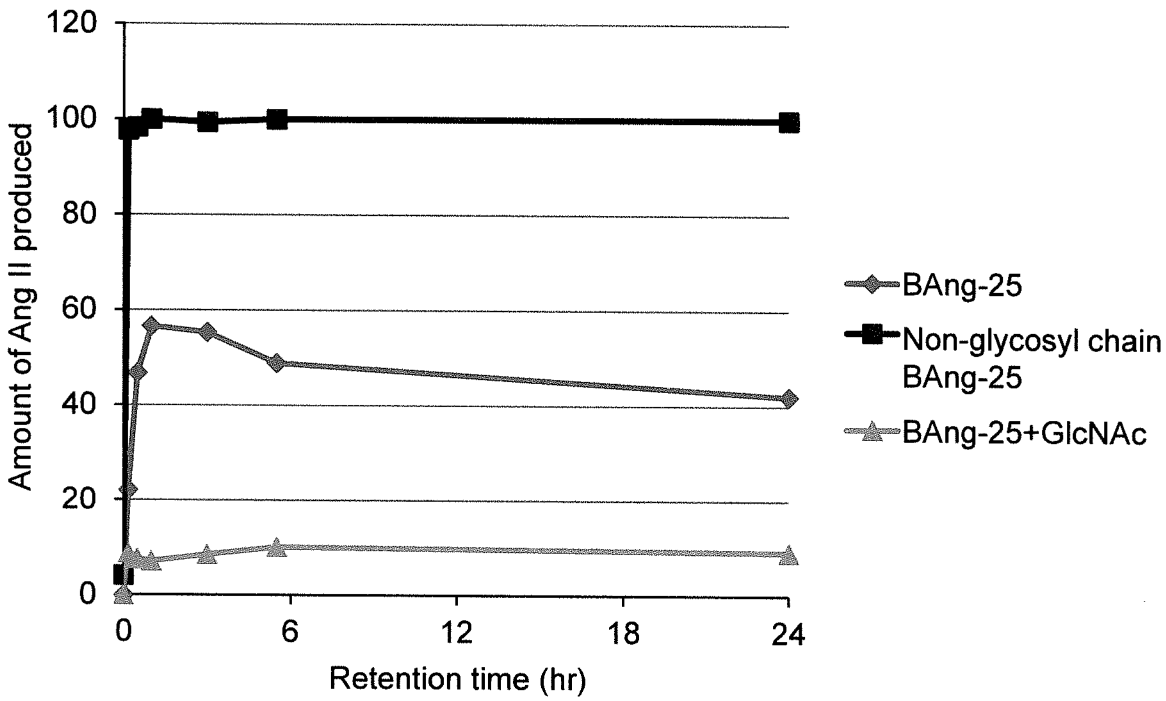
120
100
80
60
40
20
0
Amount of Ang II produced
0
6
12
18
24
Retention time (hr)
BAng-25
Non-glycosyl chain BAng-25
BAng-25+GlcNAc

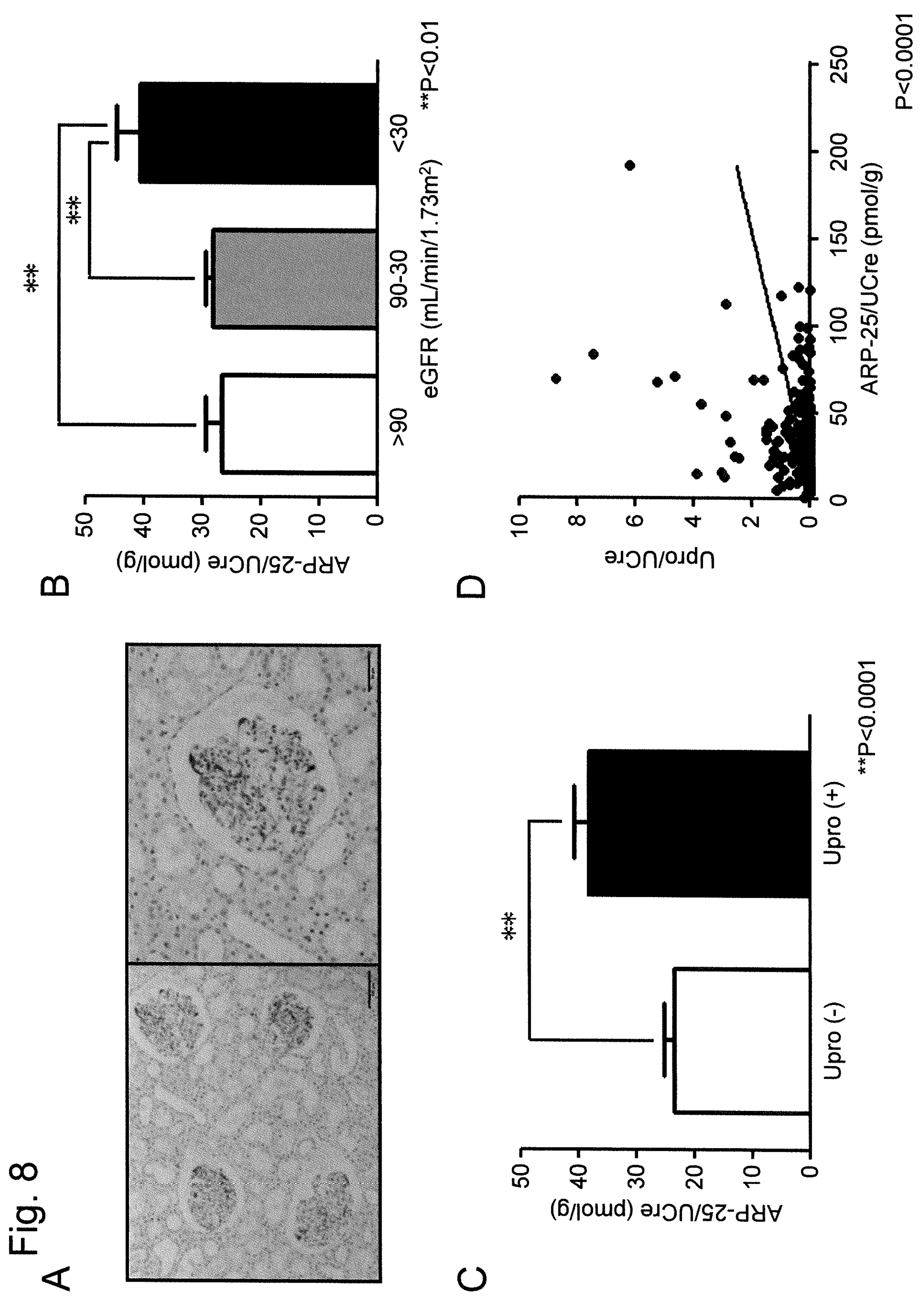
Fig. 8
A
B
ARP-25/UCre (pmol/g)
50
40
30
20
10
0
>90
90-30
<30
eGFR (mL/min/1.73m²)
**
**
**P<0.01
C
ARP-25/UCre (pmol/g)
Upro (-)
Upro (+)
**
**P<0.0001
D
Upro/UCre
10
8
6
4
2
0
50
100
150
200
250
ARP-25/UCre (pmol/g)
P<0.0001

Fig. 9

Heart (left ventricle)

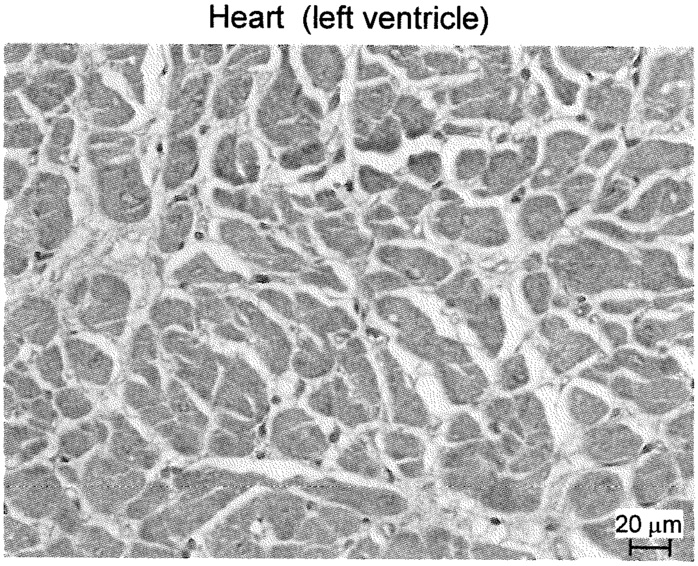

Fig. 10

Esophagus (stratified squamous epithelium)

Esophagus (smooth muscle cells)

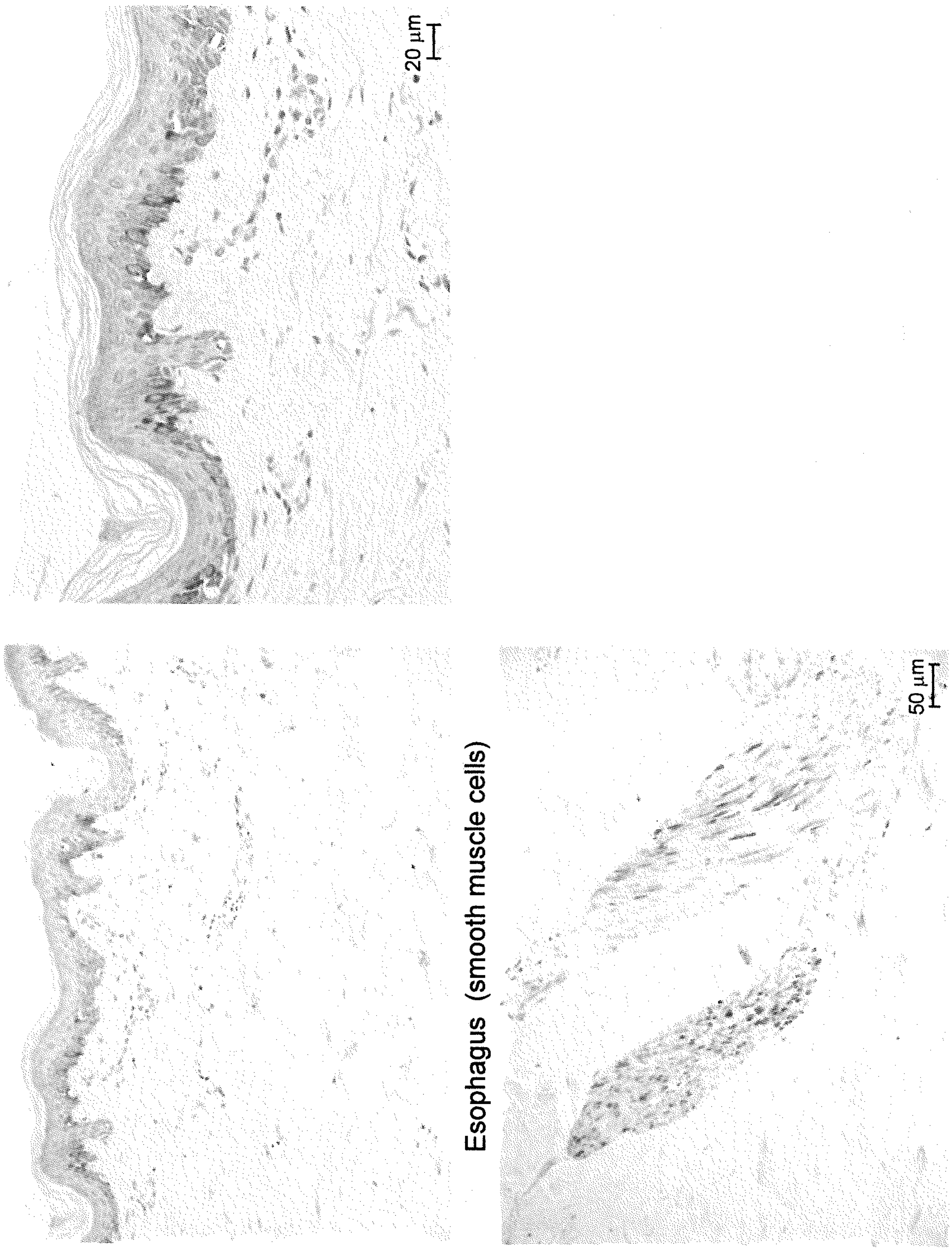

Fig. 11

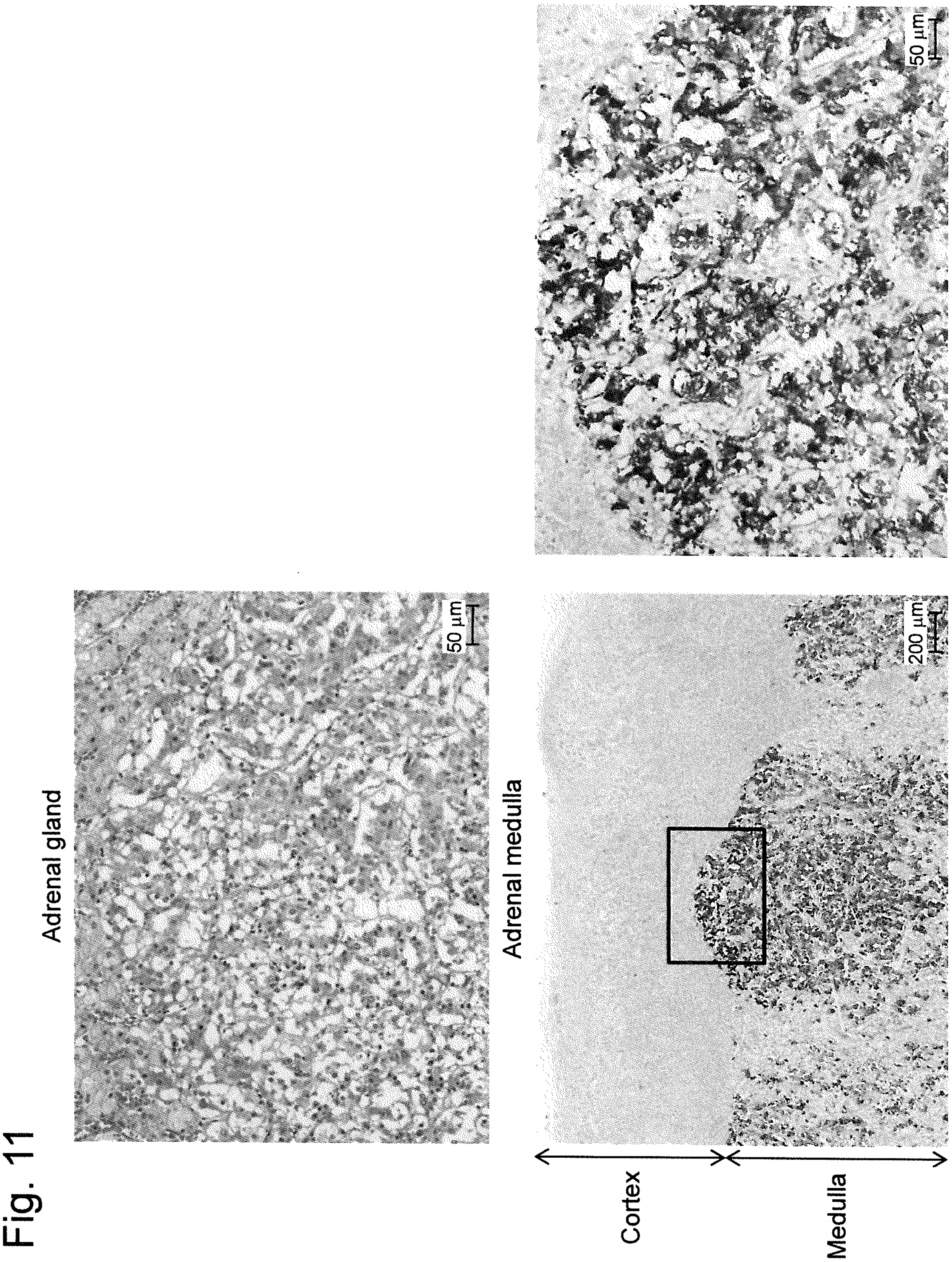
Adrenal gland
50 µm
Adrenal medulla
200 µm
50 µm
Cortex
Medulla

Fig. 12

Pancreas (islets of Langerhans)

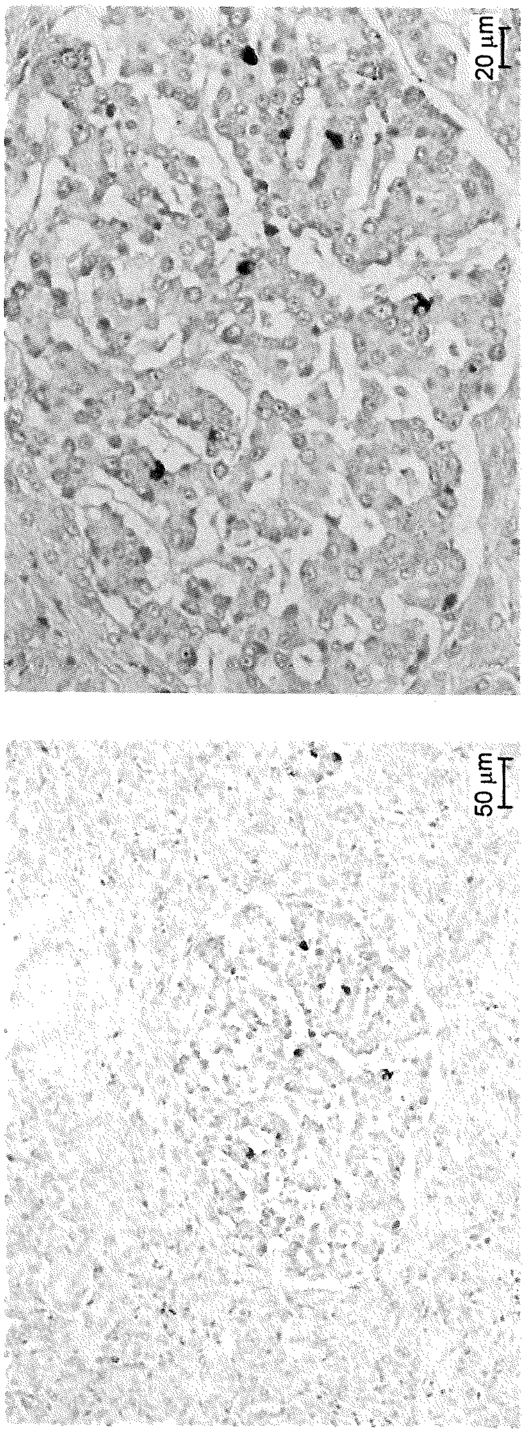

Fig. 13

Stomach (gastrointestinal endocrine cells)

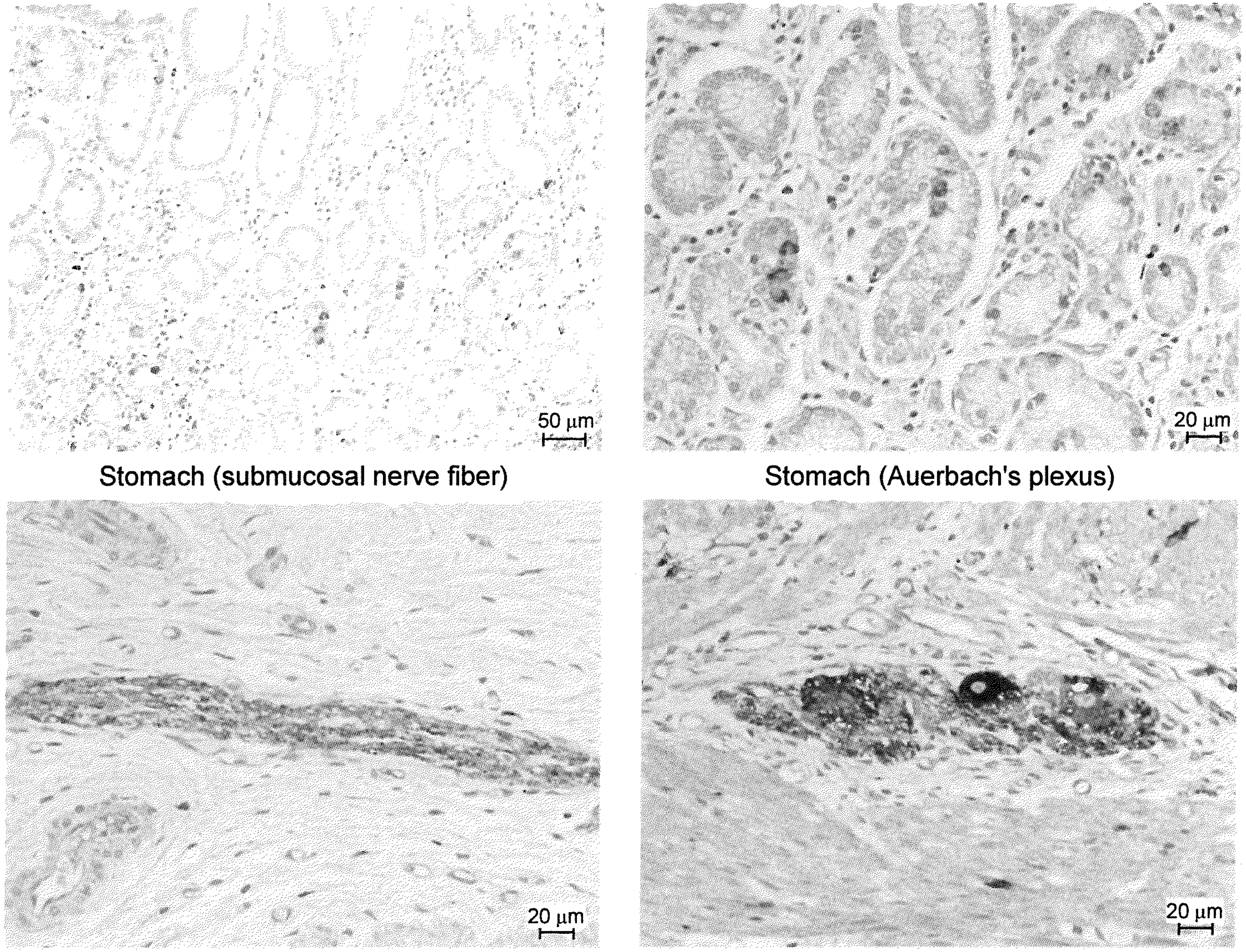


Stomach (submucosal nerve fiber)

Stomach (Auerbach's plexus)

Fig. 14

Large intestine (gastrointestinal endocrine cells)

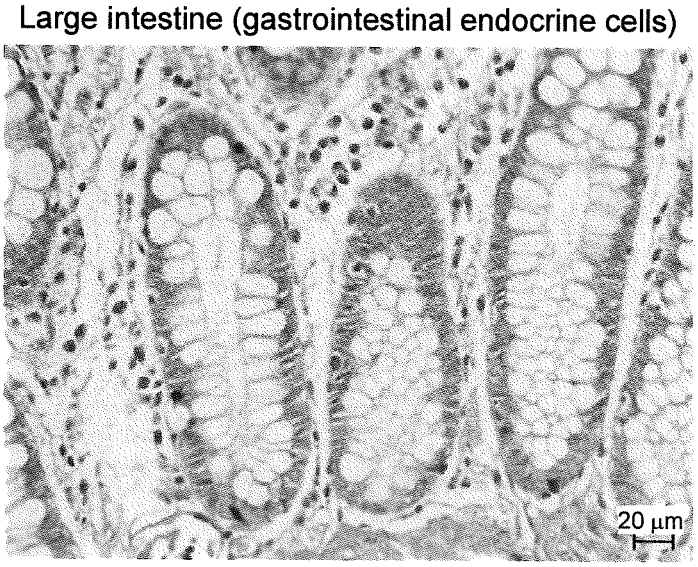

Fig. 15

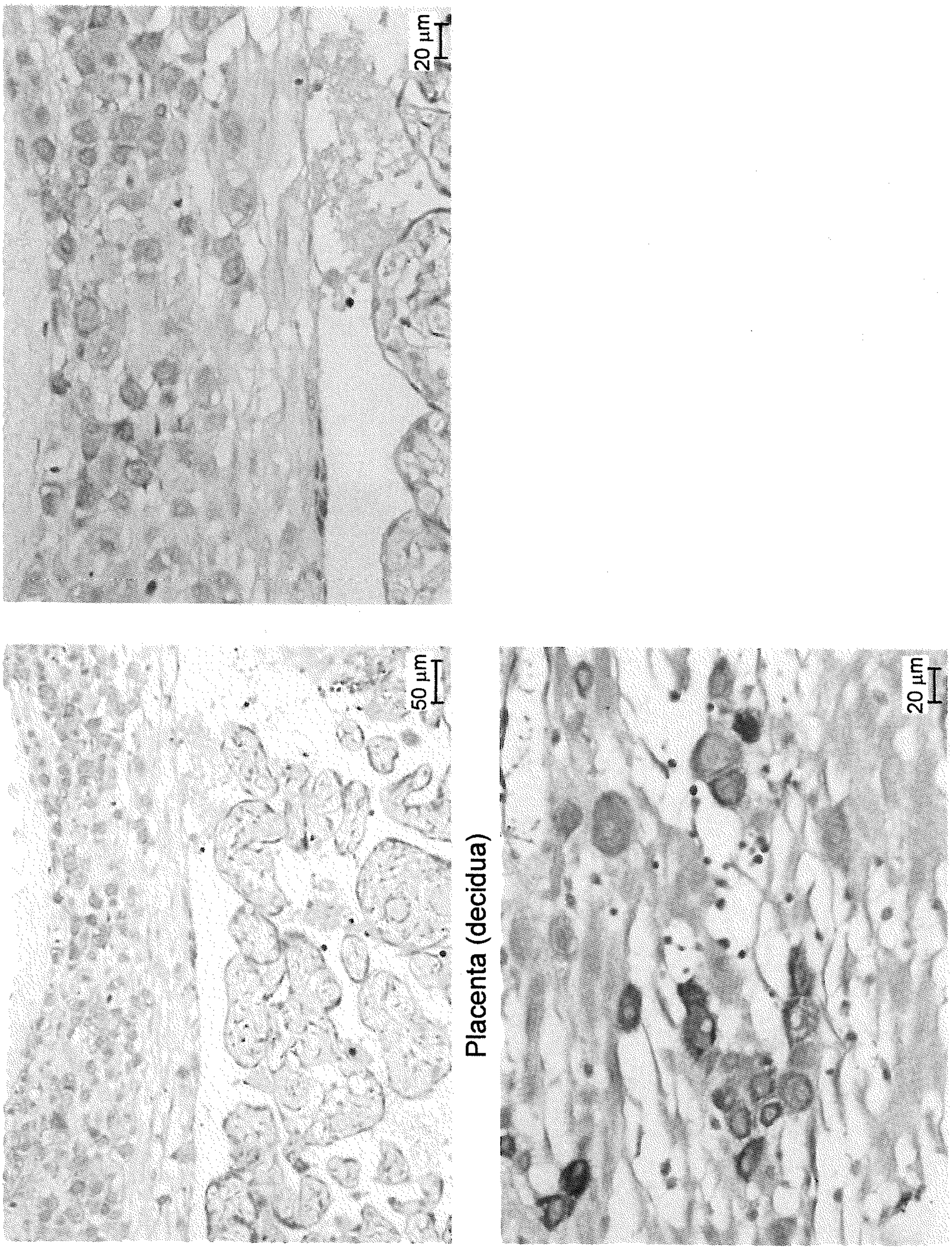


Placenta (extravillous trophoblast cells)

Placenta (decidua)

Fig. 16

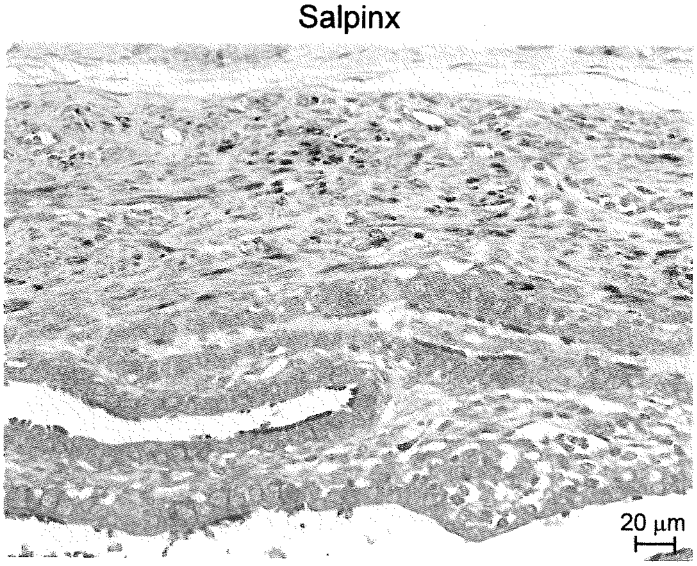

Fig. 17

Testicle

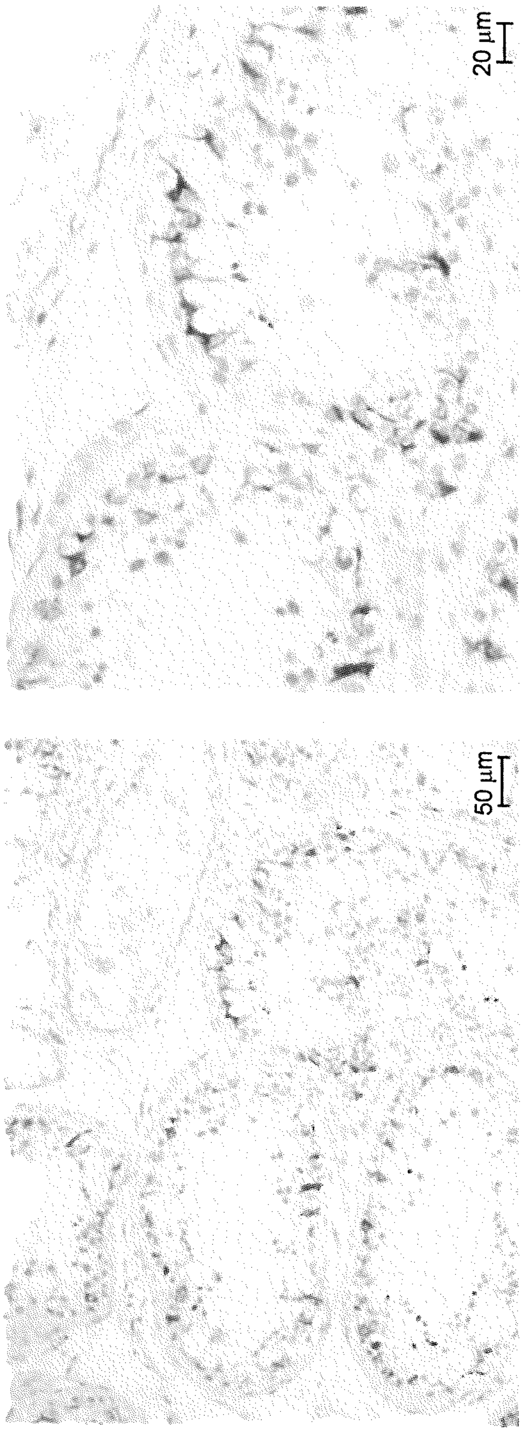

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008007482 A
- JP 2012244752 A
- JP 2013033651 A


### Non-patent literature cited in the description


- *Science,* 1974, vol. 184, 994-996
- *Am. J. Physiol.,* 1995, vol. 268, L302-308
- *Molecular and Cellular Endocrinology,* 1985, vol. 43 (1), 31-34
- *Peptides,* vol. 27 (11), 3000-3002
- *American Journal of Hypertension and Kidney Diseases,* vol. 19 (5), 541-550
- *Nature,* 1975, vol. 256, 495
- **S. NAGATA et al.** Isolation and identification of proangiotensin-12, a possible component of the renin-angiotensin system. *Biochem. Biophys. Res. Commun.,* 2006, vol. 350, 1026-1031
- **H. NAKAGAWA et al.** Identification of neutral and sialyl N-linked oligosaccharide structures from human serum glycoproteins using three kinds of high-performance liquid chromatography. *Anal. Biochem.,* 1995, vol. 226, 130-138
- **H. YAGI et al.** Development of structural analysis of sulfated N-glycans by multidimensional high performance liquid chromatography mapping methods. *Glycobiology,* 2005, vol. 15, 1051-1060

SEQUENCE LISTING

<110> University of Miyazaki

<120> Novel peptide and uses thereof

<130> PH-5739-PCT

<150> JP 2012-244752
<151> 2012-11-06

<150> JP 2013-033651
<151> 2013-02-22

<160> 1

<170> PatentIn version 3.5

<210> 1
<211> 25
<212> PRT
<213> Homo sapiens

<400> 1

Asp Arg Val Tyr Ile His Pro Phe His Leu Val Ile His Asn Glu Ser
1 5 10 15

Thr Cys Glu Gln Leu Ala Lys Ala Asn
20 25